(19) Europäisches Patentamt
European Patent Office
Office européen des brevets

(11) EP 4 684 766 A1

(12) **EUROPEAN PATENT APPLICATION**

(43) Date of publication:
28.01.2026 Bulletin 2026/05

(21) Application number: 25180521.4

(22) Date of filing: 03.06.2025

(51) International Patent Classification (IPC):
*A61F 2/915* (2013.01)    *A61F 2/07* (2013.01)
*A61F 2/958* (2013.01)

(52) Cooperative Patent Classification (CPC):
**A61F 2/915;** A61F 2/07; A61F 2/958;
A61F 2002/91575; A61F 2230/0013;
A61F 2230/0054; A61F 2230/0058;
A61F 2250/0036; A61F 2250/0082

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 24.07.2024 US 202418783153
28.04.2025 US 202519190257

(71) Applicant: Renata Medical, Inc.
Newport Beach, CA 92660 (US)

(72) Inventors:
• **ARMER, Dustin**
**Costa Mesa, 92627 (US)**
• **ABBOTT, Eason**
**Santa Monica, 90401 (US)**
• **NIA, Nima V.**
**Irvine, 92614 (US)**

(74) Representative: **Potter Clarkson**
**Chapel Quarter**
**Mount Street**
**Nottingham NG1 6HQ (GB)**

(54) **GROWTH STENT AND VALVE FOR CONGENITAL NARROWINGS**

(57) A growth stent and valve and methods for making and using the same. The growth stent and valve may be delivered to treat early-stage congenital lesions, while expanding to adult vessel diameters. In selected embodiments, the growth stent and valve can comprise a frame and may have a covering on some portion to prevent blood flow through a wall of the frame. The growth stent and valve advantageously can maintain radial strength across an entire range of diameters necessary to treat a narrowed lesion from birth and childhood through adulthood as the vessels grow over the lifetime of a patient.

Fig. 10A

## Description

FIELD

The disclosed embodiments relate to medical stents and valves and more particularly, but not exclusively, to a growth stent and valve for implantation in young patients and subsequent expansion to adult vessel sizes as the patients grow, maintaining proper strength for vessel opening throughout an entire range of expansion.

BACKGROUND

Stents have been used for many years to treat vessel disorders by allowing flow and/or preventing vessel narrowing. Narrowing vessels disrupt blood flow and can create pressure imbalances in the vasculature. Such conditions can eventually lead to serious cardiovascular compromise and/or death. For many years, the definitive treatment for such disorders was the surgical repair or replacement of the vessel segment through open heart surgery, but such surgeries are dangerous and prone to complication. Open heart surgery in neonatal and young patients may lead to negative developmental effects.

Stents have also been utilized for holding vessels open, but the growth of the patient prevents stents from being implanted at early development states as they would not be able to grow to the adult sizes. Typically, stents have specific, limited ranges of operation.

In view of the foregoing, a need exists for an improved stent and valve for implantation in young patients and subsequent expansion as the patients grow that overcomes the aforementioned obstacles and deficiencies of currently-available stents.

SUMMARY

The following numbered paragraphs are also disclosed:

1. A catheter-insertable and re-expandable flexible growth stent for implantation in a patient, comprising:

an elongated flexible stent frame comprising first, second and third annular frame strut arrangements being disposed in axial alignment between a proximal end region and a distal end region of said flexible stent frame,
the first annular frame strut arrangement having a first zigzag arrangement of first frame strut members with first peaks and first valleys,
the second annular frame strut arrangement having a second zigzag arrangement of second frame strut members with second peaks and second valleys,
the third annular frame strut arrangement being disposed between the first and second annular frame strut arrangements,
the third annular frame strut arrangement having a third zigzag arrangement of third frame strut members with:

a third peak being directly coupled with a corresponding one of the first valleys to form a first strut junction;
a fourth peak being disposed adjacent to a corresponding one of the first valleys to define a first intermediate gap;
a third valley being directly coupled with a corresponding one of the second peaks to form a second strut junction; and
a fourth valley being disposed adjacent to a corresponding one of the second peaks to define a second intermediate gap,

each of the third frame strut members associated with the first strut junction having proximal and distal end regions with respective cross-sections that are less than a cross-section of a central region of the third frame strut member,
each of the first frame strut members associated with the first strut junction having proximal and distal end regions with respective cross-sections that are less than a cross-section of a central region of the first frame strut member,
each of the third frame strut members associated with the second strut junction having proximal and distal end regions with respective cross-sections that are less than a cross-section of a central region of the third frame strut member,
each of the second frame strut members associated with the second strut junction having proximal and distal end regions with respective cross-sections that are less than a cross-section of a central region of the second frame strut member,
wherein the first and second intermediate gaps enhance an axial flexibility of said flexible stent frame,
wherein said flexible stent frame has an initial cross-section for facilitating implantation within a selected lumen of the patient, said flexible stent frame being configured to expand from the initial cross-section to a first stable expanded state for supporting the selected lumen at implantation, said flexible stent frame in the first stable expanded state defining an internal channel with a first cross-section, and
wherein said flexible stent frame is configured to subsequently expand from the first stable expanded state to a second stable expanded state as the selected lumen grows while maintaining the axial flexibility of said flexible stent frame, said flexible stent frame in the second stable expanded state continuing to support the selected lumen and defining the internal channel with a second cross-section being larger than the first cross-section.

2. The growth stent of paragraph 1, wherein each third peak of the zigzag arrangement of the third frame strut members is coupled with a respective first valley of the zigzag arrangement of the first frame strut members to form respective first strut junctions, and wherein each third valley of the zigzag arrangement of the third frame strut members is coupled with a respective second peak of the zigzag arrangement of the second frame strut members to form respective second strut junctions.

3. The growth stent of paragraph 1 or paragraph 2, wherein at least one of the first and second strut junctions comprises an arcuate member for providing the axial flexibility of said flexible stent frame.

4. The growth stent of paragraph 3, wherein the arcuate member is configured to provide a lever point for enabling said flexible stent frame to bend during implantation within the selected lumen.

5. The growth stent of paragraph 4, wherein the arcuate member comprises a c-shaped member that is configured to collapse upon itself for providing the bend in said flexible stent frame.

6. The growth stent of paragraph 4 or paragraph 5, wherein the arcuate member comprises a c-shaped member that is configured to open into a straighten member for providing the bend in said flexible stent frame.

7. The growth stent of any one of paragraph 1-6, wherein said flexible stent frame is made from cobalt chromium.

8. The growth stent of any one of paragraph 1-7, further comprising a covering for enclosing a periphery of said flexible stent frame.

9. The growth stent of paragraph 8, wherein said covering comprises a fluid-impermeable covering for preventing leaks due to ingrowth of the selected lumen as the patient grows.

10. The growth stent of any one of paragraph 1-9, wherein the initial first cross-section is less than or equal to eight millimeters.

11. The growth stent of any one of paragraph 1-10, wherein the first cross-section is between eight millimeters and ten millimeters.

12. The growth stent of any one of paragraph 1-11, wherein the wherein the second cross-section is between fifteen millimeters and twenty millimeters.

BRIEF DESCRIPTION OF THE DRAWINGS

Fig. 1A is an exemplary detail diagram illustrating an exemplary embodiment of a growth stent in a first expanded state.
Fig. 1B is an exemplary detail diagram illustrating an alternative embodiment of the growth stent of Fig. 1A, wherein the growth stent is shown in a second expanded state.
Fig. 2A is an exemplary detail diagram illustrating an alternative embodiment of the growth stent of Figs. 1A-B, wherein the growth stent is shown in an initial state for implantation.
Fig. 2B is an exemplary detail diagram illustrating another alternative embodiment of the growth stent of Figs. 1A-B, wherein the growth stent is shown in an expanded state for defining an internal channel with a first predetermined cross-section.
Fig. 2C is an exemplary detail diagram illustrating yet another alternative embodiment of the growth stent of Figs. 1A-B, wherein the growth stent is shown in an expanded state for defining the internal channel with a second predetermined cross-section.
Fig. 2D is an exemplary detail diagram illustrating still another alternative embodiment of the growth stent of Figs. 1A-B, wherein the growth stent is shown in an expanded state for defining the internal channel with a third predetermined cross-section.
Fig. 3A is an exemplary detail diagram illustrating another alternative embodiment of the growth stent of Figs. 1A-B, wherein the internal channel comprises an open channel.
Fig. 3B is an exemplary detail diagram illustrating an alternative embodiment of the growth stent of Fig. 3A, wherein the growth stent includes one or more leaflets extending into the internal channel.
Fig. 4A is an exemplary detail diagram illustrating further alternative embodiment of the growth stent of Figs. 1A-B, wherein the growth stent includes a plurality of arcuate members.
Fig. 4B is an exemplary detail diagram illustrating further alternative embodiment of the growth stent of Fig. 4A, wherein pairs of the arcuate members cooperate.
Fig. 5A is an exemplary detail diagram illustrating an alternative embodiment of the growth stent of Figs. 4A-B, wherein the growth stent is shown in an initial state for implantation.
Fig. 5B is an exemplary detail diagram illustrating another alternative embodiment of the growth stent of Figs. 4A-B, wherein the growth stent is shown in an expanded state for defining an internal channel with a first predetermined cross-section.

Fig. 5C is an exemplary detail diagram illustrating yet another alternative embodiment of the growth stent of Figs. 4A-B, wherein the growth stent is shown in an expanded state for defining the internal channel with a second predetermined cross-section.

Fig. 5D is an exemplary detail diagram illustrating still another alternative embodiment of the growth stent of Figs. 4A-B, wherein the growth stent is shown in an expanded state for defining the internal channel with a third predetermined cross-section.

Fig. 6A is an exemplary detail diagram illustrating a two-dimensional flat view of the growth stent of Figs. 4A-B.

Fig. 6B is an exemplary detail diagram illustrating a side view of the growth stent of Fig. 6A.

Fig. 7A is exemplary detail diagram illustrating an embodiment of a catheter delivery system for implanting the growth stent of Figs. 1A-B.

Fig. 7B is exemplary detail diagram illustrating an alternative embodiment of the catheter delivery system of Fig. 7A, wherein the catheter delivery system is shown in a closed configuration.

Fig. 8A is exemplary detail diagram illustrating another alternative embodiment of the catheter delivery system of Figs. 7A-B, wherein a delivery catheter of the catheter delivery system is disposed adjacent to an implantation site.

Fig. 8B is exemplary detail diagram illustrating an alternative embodiment of the catheter delivery system of Fig. 8A, wherein the catheter delivery system is shown in an open configuration.

Fig. 8C is exemplary detail diagram illustrating an alternative embodiment of the catheter delivery system of Fig. 8B, wherein the catheter delivery system expands the growth stent from an initial state to an expanded state.

Fig. 8D is exemplary detail diagram illustrating an alternative embodiment of the catheter delivery system of Fig. 8C, wherein the catheter delivery system is withdrawn from the implantation site, leaving the expanded growth stent.

Fig. 9 is an exemplary flow chart illustrating an embodiment of a method for implanting of the growth stent of Figs. 1A-B via a balloon catheter delivery system.

Fig. 10A is an exemplary detail diagram illustrating yet another alternative embodiment of the growth stent of Figs. 1A-B, wherein the growth stent includes a plurality of growth cell members utilizing non-connected junctions for flexibility and stent retention.

Fig. 10B is an exemplary detail diagram of the embodiment of Fig. 10A.

Fig. 11A is a detail drawing illustrating an exemplary embodiment of a growth cell member for the growth stent of Figs. 10A-B, wherein a first pair of coupled growth cell struts of the growth cell member is coupled with a second pair of coupled growth cell struts to form a growth cell junction.

Fig. 11B is a detail drawing illustrating an exemplary alternative embodiment of the growth cell member of Fig. 11A, wherein the first pair of coupled growth cell struts of the growth cell member is separate from the second pair of coupled growth cell struts to define an intermediate gap.

Fig. 12 is an exemplary detail diagram illustrating still another alternative embodiment of the growth stent of Figs. 1A-B, wherein the growth stent of Figs. 1A-B includes a stent frame utilizing non-connected junctions and reduced junctions for increased flexibility and stent retention.

Fig. 13A is a detail drawing illustrating an exemplary embodiment of a growth cell member for the growth stent of Fig. 12, wherein a first pair of coupled growth cell struts of the growth cell member is coupled with a second pair of coupled growth cell struts to form a growth cell junction.

Fig. 13B is a detail drawing illustrating an exemplary alternative embodiment of the growth cell member of Fig. 13A, wherein the first pair of coupled growth cell struts of the growth cell member is separate from the second pair of coupled growth cell struts to define an intermediate gap.

It should be noted that the figures are not drawn to scale and that elements of similar structures or functions may be generally represented by like reference numerals for illustrative purposes throughout the figures. It also should be noted that the figures are only intended to facilitate the description of the preferred embodiments. The figures do not illustrate every aspect of the described embodiments and do not limit the scope of the present disclosure.

DETAILED DESCRIPTION OF THE PREFERRED EMBODIMENTS

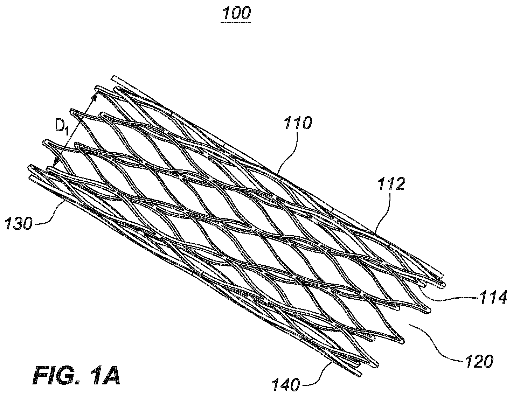

Since currently-available stents have specific, limited ranges of operation and, when implanted at early development states are unable to grow to adult sizes, an expandable growth stent and valve that can maintain proper strength throughout a predetermined expansion range can prove desirable and provide a basis for a wide range of system applications, such as implantation in neonates and other young patients and subsequent expansion as the patients grow. This result can be achieved, according to selected embodiments disclosed herein, by a growth stent 100 as illustrated in Figs. 1A-B. The growth stent 100, in selected embodiments, can be utilized to provide positive results in pediatric patients, such as neonates, infants, toddlers, small children, children, and all manner of young persons, without limitation. For example, the growth stent 100 advantageously can be applied for treatment of all manner of congenital heart diseases that involve blood vessel narrowing, deformities, or other illnesses, and as well as many other manners of body tissue in tubular form, like an esophagus.

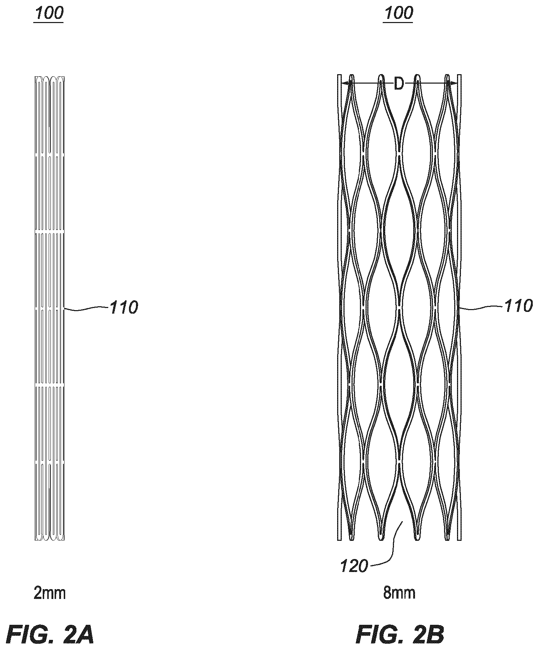

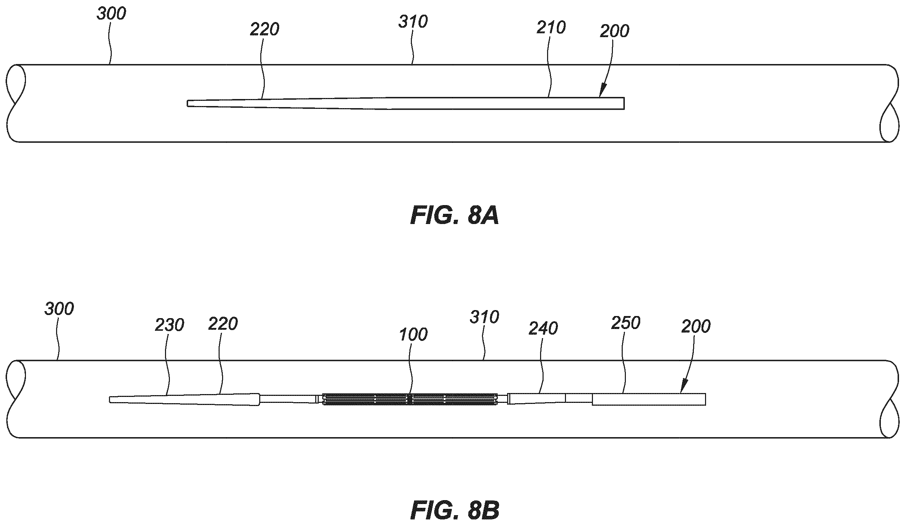

Turning to Fig. 1A, the growth stent 100 is shown as including an elongated stent frame (or body) 110 for forming an internal channel 120. The growth stent 100 can be implanted within a vessel (or lumen) 300 (shown in Figs. 8A-D) of a patient to hold open or otherwise reinforce the lumen 300. The stent body 110 can be provided in an initial (or unexpanded or crimped) state (or cross-section) as shown in Fig. 2A. In the initial state, the stent body 110 can have any predetermined initial size, shape, diameter, cross-section and/or other dimension for facilitating insertion of the growth stent 100 into the lumen 300. The initial size, shape, diameter, cross-section and/or other dimension D of the stent body 110 can be application dependent.

Upon being positioned at a selected implantation site 310 (or other area of interest) (shown in Figs. 8A-D) within the lumen 300, the stent body 110 can be expanded from the initial state to a first expanded state as shown in Fig. 1A. In the first expanded state, the stent body 110 can have a first predetermined expanded size, shape, diameter, cross-section and/or other dimension D. The stent body 110, when expanded in the first expanded state, can define the internal channel 120. The internal channel 120 can have a first predetermined diameter, cross-section and/or other dimension Di for facilitating an unobstructed (or normal) flow of a bodily fluid, such as blood, bile or other luminal liquids, through the lumen 300 via the growth stent 100.

In selected embodiments, the expanded stent body 110 can comprise a body wall 112 that defines the internal channel 120. The body wall 112, for example, can include an inner surface 114 for defining the internal channel 120. The internal channel 120 preferably extends from a proximal end region 130 of the stent body 110 to a distal end region 140 of the stent body 110 as illustrated in Fig. 1A. The growth stent 100 thereby can enable the bodily fluid to flow, preferably unobstructed, from the proximal end region 130 to the distal end region 140 via the internal channel 120. Stated somewhat differently, the growth stent 100 can be provided in generally tubular shape.

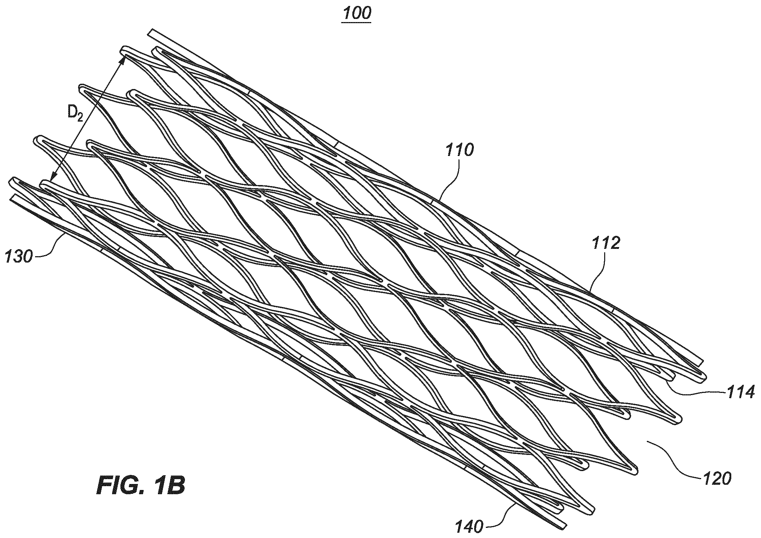

Advantageously, the stent body 110 can be further expanded from the first expanded state to a second expanded state as shown in Fig. 1B. The stent body 110 can be expanded to the second expanded state based upon one or more preselected criteria. Exemplary preselected criteria can include, but are not limited to, a preselected duration after implantation of the growth stent 100, a condition of the lumen 300 and/or growth (or other change in size or other condition) of the patient. In the second expanded state, the stent body 110 can have a second predetermined expanded size, shape, diameter, cross-section and/or other dimension, which is greater than the first predetermined expanded size, shape, diameter, cross-section and/or other dimension of the stent body 110.

The stent body 110, when expanded in the second expanded state, can define the internal channel 120 with a second predetermined diameter, cross-section and/or other dimension $D_2$ for maintaining the unobstructed flow of bodily fluid through the lumen 300 via the further-expanded growth stent 100. Stated somewhat differently, the stent body 110 can be provided with a tubular shape and/or can have relatively wide securing end portions (not shown) at the proximal and distal end regions 130, 140 when expanded in the second expanded state. Although shown and described as having two expanded states with reference to Figs. 1A-B for purposes of illustration only, the growth stent 100 can support any predetermined number of expanded states. The stent body 110, when expanded in a selected expanded state, can define the internal channel 120 with a respective predetermined diameter, cross-section and/or other dimension for maintaining the unobstructed flow of bodily fluid through the lumen 300 via the growth stent 100. The predetermined number of expanded states and the associated predetermined diameters, cross-sections and/or other dimensions, for example, can be application-dependent.

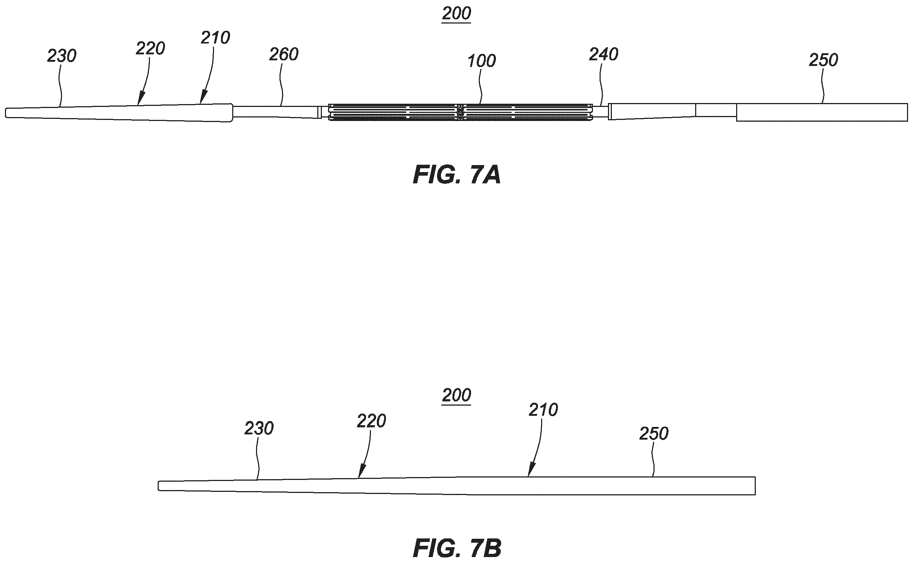

The growth stent 100 advantageously can be expanded to cover the wide range of anatomical diameters necessary to treat narrowed lesions across an entire lifetime of the patient. Stated somewhat differently, the predetermined number of expanded states supported by the growth stent 100 can configure the growth stent 100 to provide a wide range of anatomical diameters. The growth stent 100, for example, can be crimped on the delivery catheter 210 at small enough sizes for a neonatal patient, such as considerably less than an 8 French crimped growth stent 100, with an ability to be expanded later to teen and/or adult sizes of greater than 18mm in diameter. In selected embodiments, the growth stent 100 can be crimped or otherwise disposed on a delivery catheter 210 (shown in Figs. 7A-B) with suitable predetermined diameter, cross-section and/or other dimension such as three French, four French, five French or smaller.

Figs. 2A-D illustrate selected embodiments of a growth stent frame at various cycles throughout a patient's life, as the diameter of a vessel (or lumen) 300 (shown in Figs. 8A-D) of the patient grows. In this specific example, the growth stent frame 110 can maintain strength at the various diameters. Turning to Fig. 2A, the growth stent 100 is shown in the initial state for implantation. The stent body 110, in the initial state, can have a predetermined initial size, shape, diameter, cross-section or other dimension of, for example, 2mm for facilitating insertion of the growth stent 100 into the lumen 300.

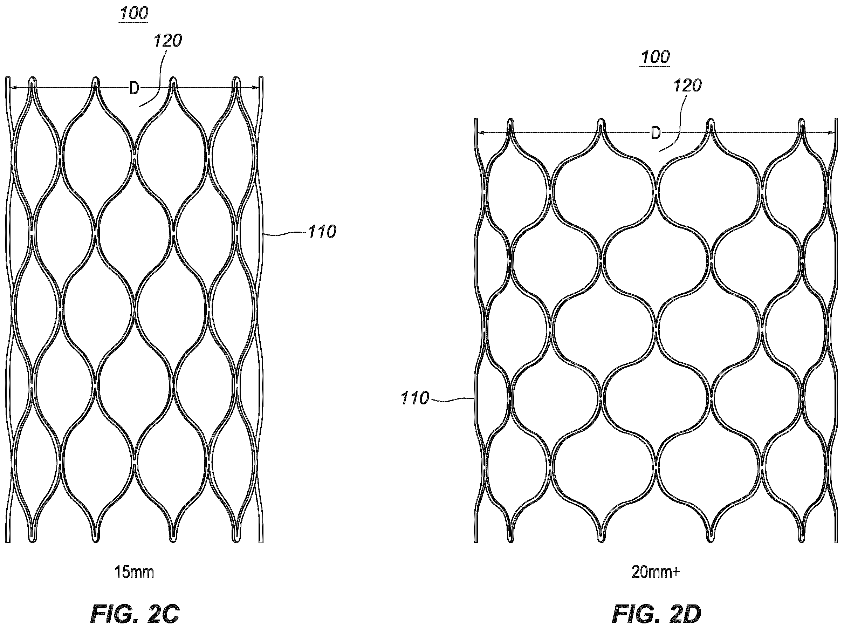

When the growth stent 100 is in an expanded state, the stent body 110 can define the internal channel 120 with any suitable predetermined diameter, cross-section or other dimension D. The stent body 110, for example, can define the internal channel 120 with a first predetermined diameter, cross-section or other dimension D of 8mm as shown in Fig. 2B, a second predetermined diameter, cross-section or other dimension D of 15mm as shown in Fig. 2C and/or a third predetermined diameter, cross-section or other dimension D of 20mm or more as shown in Fig. 2D, without limitation. The growth stent 100 thereby can be provided to hold open a narrow vessel 300 (shown in Figs. 8A-D) and allow for normal flow of blood or other bodily fluids.

Advantageously, the growth stent 100 can support an extended expansion ratio. The growth stent 100, in selected embodiments, can support expansion to an expanded state with a predetermined diameter, cross-section or other dimension D that is between five times and fifteen times the diameter, cross-section or other dimension D of the growth stent 100 in the initial state, including any expansion sub-ranges, such as a sub-range with a multiple of five (*i.e.*, between five times and ten times) and/or a sub-range with a multiple of ten (*i.e.*, between five times and fifteen times), within the selected range of angles, without limitation. The second predetermined diameter, cross-section or other dimension D of the growth stent 100, for example, can be between five and six-tenths times and seven and one-half times of the diameter, cross-section or other dimension D of the growth stent 100 in the initial state in selected embodiments.

As an example, the growth stent 100 can be implanted in a vessel (or lumen) 300 (shown in Figs. 8A-D) of an infantile patient through expansion to a diameter of less than 10mm and have proper strength to be expanded over the course of a patient's lifetime to significantly greater than 18mm. The exemplary growth stent 100 can be tracked through the vessel 300 of the infantile patient via a delivery catheter 210 of a catheter delivery system 200 (collectively shown in Figs. 7A-B) that is considerably less than 8 French in diameter and, in some embodiments, less than five French in diameter and beyond. Other exemplary diameters of the catheter delivery system 200 can include, but are not limited to, three French or four French.

The growth stent 100, additionally and/or alternatively, may be implanted at a variety of sizes and then later re-expanded after the pediatric patient grows. While delivered on a delivery catheter 210 (shown in Figs. 7A-B) with a predetermined diameter, cross-section and/or other dimension of 2mm or less, in some examples, the expansion of the growth stent 100 at implantation may be within any suitable range for a pediatric patient. Although the most common expansion range is less than 12mm, the growth stent 100 typically can be expanded to a diameter, cross-section and/or other dimension that is between 6mm and 15mm, without limitation. The growth stent 100 may be later re-expanded (or re-dilated) to any suitable diameter, cross-section and/or other dimension after the pediatric patient grows. An expansion catheter system, for example, can be introduced into the patient such that a stent expansion system 260 (shown in Fig.

7A), such as a balloon, is disposed within the channel 120 formed by the stent body 110 of the growth stent 100. In selected embodiments, the re-expanded diameter, cross-section and/or other dimension of the growth stent 100 can be based upon an expanded size of the stent expansion system 260.

Being configured to operate at any suitable diameter while maintaining structural integrity and be re-dilated for growth of the patient, the growth stent 100 can be expanded and/or re-expanded to any size that is less than the maximum stent diameter. Exemplary maximum diameters can be within a range between 20mm and 30mm, without limitation. In selected embodiments, the maximum diameter can be 26mm. The growth stent 100 thereby can be expanded and/or re-expanded to fits a size of an adult blood vessel. The growth stent 100 optionally may be delivered on a compliant or semi-compliant balloon (not shown). Therefore, the growth stent 100 may be expanded and/or re-expanded to diameters, cross-sections and/or other dimensions with the precision of as small as 0.1mm.

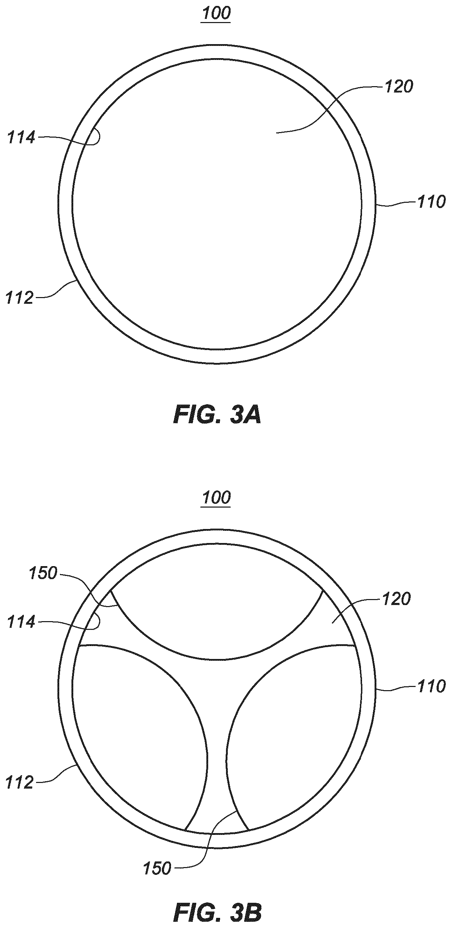

If loaded on a 6mm balloon, for example, a growth stent 100 may be implanted at 5.7mm, or in some cases 5.8mm, or in other cases 6.2mm, with every decimal achievable as the stent maintains strength at all sizes. By maintaining a radial strength over its operating range, the growth stent 100 advantageously may be implanted at and/or re-expanded to diameters of 1mm, 2mm, 3mm, 4mm, 5mm, 6mm, 7mm, 8mm, 9mm, 10mm, 11mm, 12mm, 13mm, 14mm, 15mm, 16mm, 17mm, 18mm, 19mm, 20mm, 21mm, 22mm, 23mm, 24mm, 25mm, 26mm, and/or any decimal (or fraction) in between the listed diameters. As the growth stent 100 may operate at any one of these sizes, the stent expansion system 260 can be inflated or otherwise expanded to an initial diameter of 6mm with continual re-dilations to 8mm, 10mm, 12mm, 14mm, 16mm, 18mm, 20mm, and finally 22mm in selected embodiments. This pattern of inflation and re-expansion likewise can apply to a growth stent 100 with any of the expansion ranges set forth herein. When provided with the tubular shape, the channel 120 formed by the stent body 110 can comprise an open channel in the manner illustrated in Fig. 3A. The growth stent 100 optionally can support a valvular function. In selected embodiments, for example, the growth stent 100 can include one or more leaflets 150 as shown in Fig. 3B. The leaflets 150 can be attached to the inner surface 114 of the body wall 112 and extend into the channel 120 formed by the stent body 110. The leaflets 150 thereby can provide the valvular function via the growth stent 100.

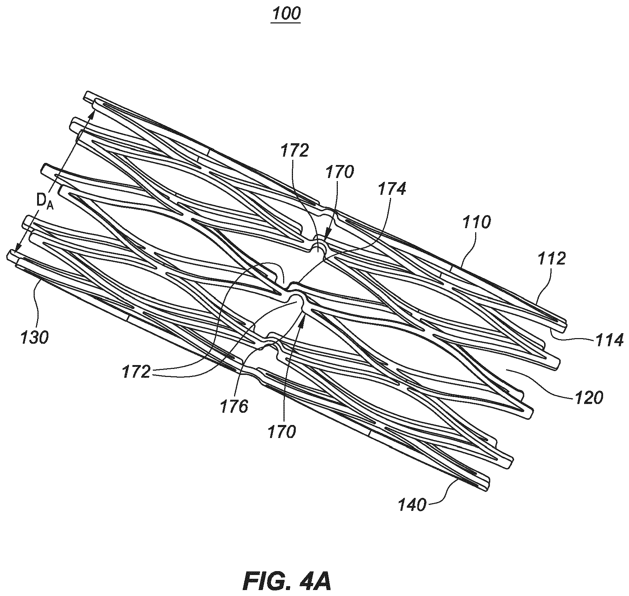

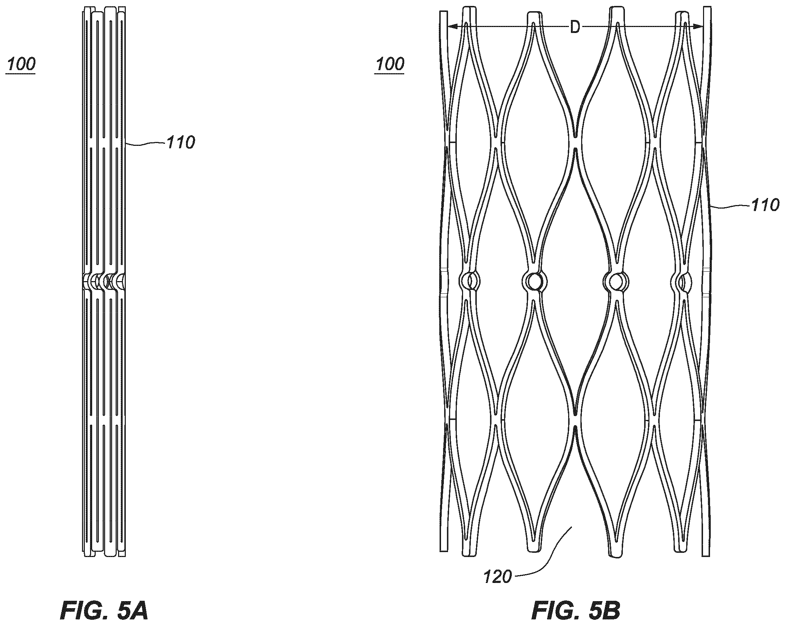

An alternative embodiment of the growth stent 100 is shown in Figs. 4A-B. Turning to Fig. 4A, the growth stent 100 is shown as including an elongated stent frame (or body) 110 for forming an internal channel 120. In the manner discussed in additional detail with reference to Figs. 1A-B, the growth stent 100 can be implanted within a vessel (or lumen) 300 (shown in Figs. 8A-D) of a patient to hold open or otherwise reinforce the lumen 300. The stent body 110 can be provided in an initial (or unexpanded or crimped) state as shown in Fig. 5A. In the initial state, the stent body 110 can have any predetermined initial size, shape, diameter, cross-section and/or other dimension for facilitating insertion of the growth stent 100 into the lumen 300. The initial size, shape, diameter, cross-section and/or other dimension D of the stent body 110 can be application dependent.

Upon being positioned at a selected implantation site 310 (or other area of interest) (shown in Figs. 8A-D) within the lumen 300, the stent body 110 can be expanded from the initial state to a first expanded state as shown in Fig. 5B. In the first expanded state, the stent body 110 can have a first predetermined expanded size, shape, diameter, cross-section and/or other dimension D. The stent body 110, when expanded in the first expanded state, can define the internal channel 120. The internal channel 120 can have a first predetermined diameter, cross-section and/or other dimension D for facilitating an unobstructed (or normal) flow of a bodily fluid, such as blood, bile or other luminal liquids, through the lumen 300 via the growth stent 100.

In selected embodiments, the expanded stent body 110 can comprise a body wall 112 that defines the internal channel 120 that preferably extends from a proximal end region 130 of the stent body 110 to a distal end region 140 of the stent body 110 as illustrated in Fig. 4A. The growth stent 100 thereby can enable the bodily fluid to flow, preferably unobstructed, from the proximal end region 130 to the distal end region 140 via the internal channel 120. Stated somewhat differently, the growth stent 100 can be provided in generally tubular shape and/or can support a valvular function in the manner discussed herein with reference to Figs. 3A-B.

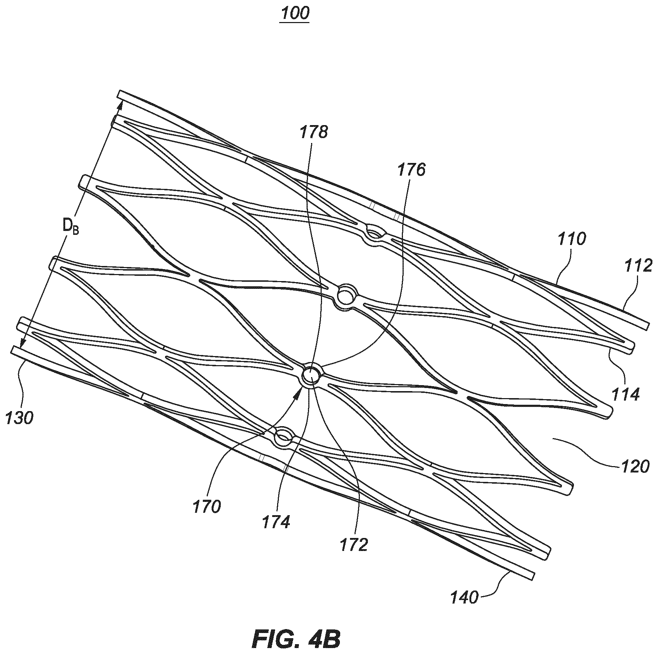

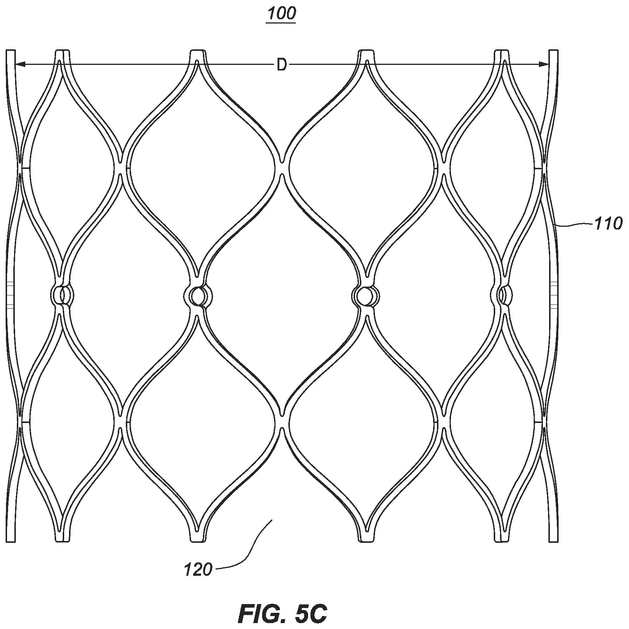

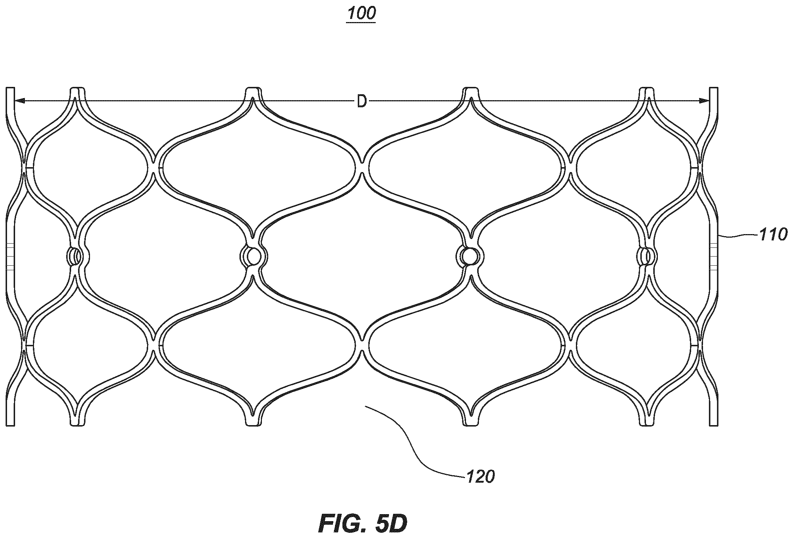

As shown in Fig. 4A, one or more selected junctions 170 of the stent body (or frame) 110 can be formed from frame members that include respective arcuate members, such as arcuate members 174, 176. The arcuate members 174, 176 each can define a central recess 172. The growth stent 100 can expand in the manner discussed herein such that the diameter, cross-section and/or other dimension D of the internal channel 120 can increase from a smaller dimension $D_A$ as shown in Fig. 4A to a larger dimension $D_B$ as shown in Fig. 4B in the manner discussed herein. The expansion of the growth stent 100 can comprise expansion between any two predetermined states. For example, the expansion of the growth stent 100 can comprise expansion from the initial state illustrated in Fig. 5A to the first expanded state illustrated in Fig. 5B, expansion from the first expanded state illustrated in Fig. 5B to the second expanded state illustrated in Fig. 5C and/or expansion from the second expanded state illustrated in Fig. 5C to the third expanded state illustrated in Fig. 5D, or any other expansion of the growth stent 100 without limitation.

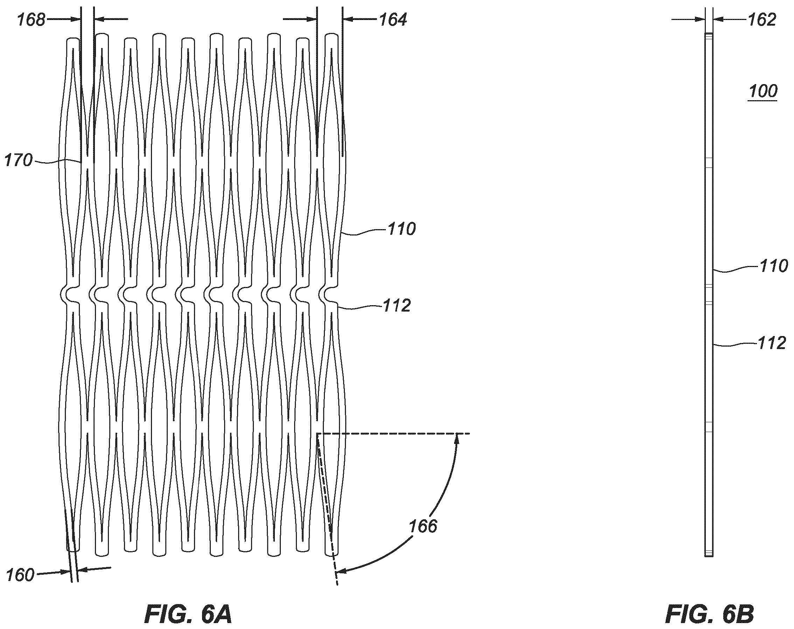

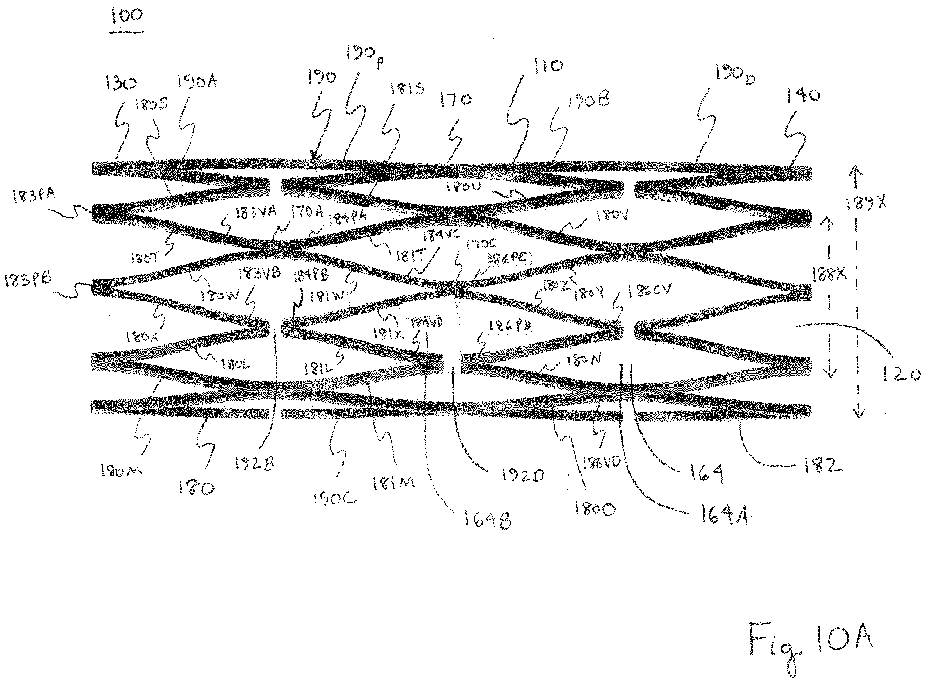

The arcuate members 174, 176 advantageously can provide axial flexibility for the growth stent frame 110, for example, if the arcuate members 174, 176 are thinner than a strut width 160 (shown in Fig. 6A) of a strut (or strut member) 180 (shown in Figs. 10A-B) of the growth stent 100. The arcuate members 174, 176 can be provided with any suitable shape, size or other dimension. In some embodiments, the arcuate members 174, 176 can utilize a rounded "c-like" shape (or "u-like" shape) to allow deformation that is opposite of the usual column strength provided by the growth stent frame 110. The arcuate members 174, 176 can provide a lever point in the structure of the frame 110, potentially allowing for a bend in the stent frame 110 when tracking to a target location or potentially after deployment, depending on the configuration needed for either. The bend can occur by the "c-like" shape deforming to collapse upon itself and/or to open to be more of a straight line, depending on the direction of deformation.

Additionally and/or alternatively, the arcuate members 174, 176 can be provided with a "v-like" shape or other pointed (or sharp) shape. Arcuate members 174, 176 with a "v-like" shape may take less force to deform, bend and/or collapse than arcuate members 174, 176 with a "c-like" shape. In selected embodiments, the growth stent 100 can comprise any suitable arrangement of one or more arcuate members 174, 176 with "v-like" shapes and/or one or more arcuate members 174, 176 with "c-like" shapes to offer multiple deforming areas in a single junction 170 (shown in Figs. 4A and 6A). The arcuate members 174, 176, for instance, can be arranged with a uniform orientation in the manner illustrated in Figs. 4A-B and/or with non-uniform orientations. Exemplary non-uniform orientations can include, but are not limited to, selected arcuate members 174, 176 having a first orientation that is opposite a second orientation associated with other arcuate members 174, 176. The arcuate members 174, 176, in selected embodiments, can be arranged such that the arcuate members 174, 176 have alternating orientations around a periphery 182 (shown in Figs. 10A-B) of the growth stent frame 110. Other embodiments of the growth stent 100 may contain joint connections in a variety of other designs such as meandering struts and/or narrowed struts. In selected embodiments, the of the growth stent 100 may have weakened joints and/or pre-cut joints that are deformable and/or breakable around a bend.

In selected embodiments, one or more junctions 170 (shown in Figs. 4A and 6A) can be omitted or otherwise eliminated from the growth stent frame 110 of the growth stent 100 to provide a flexible stent frame. The growth stent frame 110, in other words, can eliminate one or more junctions 170 disposed between adjacent cells 164 (shown in Fig. 6A) of the struts 180 (shown in Figs. 10A-B). Elimination of the junctions 170 advantageously can help provide flexibility and other benefits to the growth stent 100. In selected embodiments, the flexible stent frame can be provided by eliminating the junctions 170 from an otherwise rigid growth stent frame 110. Additionally and/or alternatively, the growth stent frame 110 can comprise a partially-flexible growth stent frame, and elimination of the junctions 170 can help to enhance the flexibility of the flexible stent frame.

In selected embodiments, eliminating the junctions 170 can enable increased concentration of strain on remaining junctions 170 from outside forces, allowing for the junctions 170 to act as hinge points and creating flexibility within the stent frame 110. Open cells 164A (shown in Figs. 10A-B) without junctions 170 can flex outwardly and/or inwardly around a bend as well, allowing the apices of the cells 164 to point away from a lumen of the stent frame 110 and/or allowing for increased apical engagement in vasculature of the patient. In the presence of short-length and acute radial force, open cells 164A without junctions 170 may displace more easily than closed cells 164B (shown in Figs. 10A-B) with connected junctions 170, providing varying engagement with contours of curved vasculature in contrast to a straight lumen.

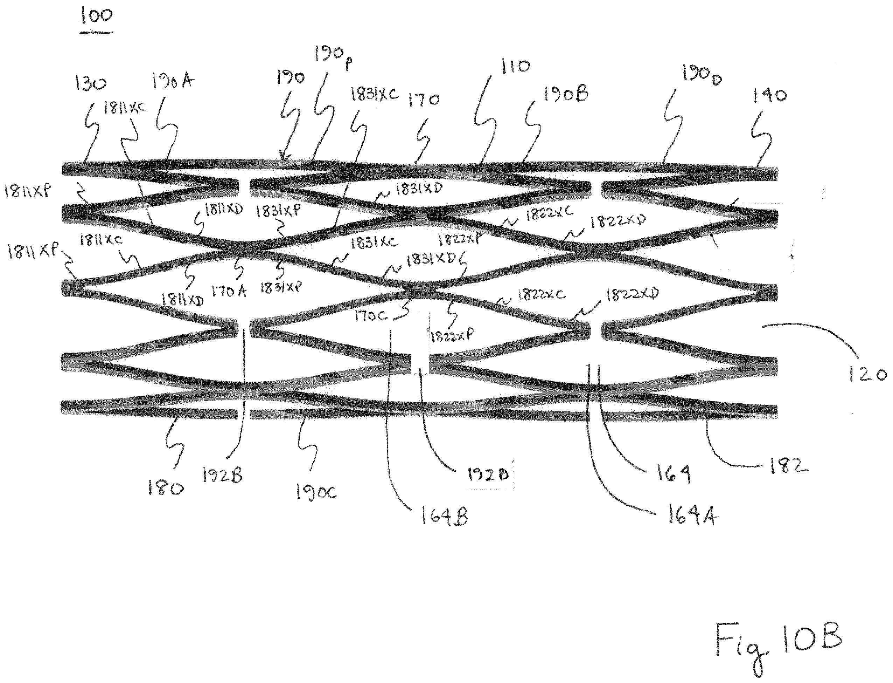

Other embodiments of the stent frame 110 may have two or more rows of struts 180 that have a uniform number of connected junctions 170 and/or two or more rows of struts 180 that have different numbers of connected junctions 170. For example, a middle row of the stent (or valve) frame 110 may have more junction 170 connections than an outer row(s) of the stent frame 110, enabling more flexibility and/or conformability with a vessel of a patient at proximal and distal end regions 130, 140 (shown in Figs. 1A-B and 10) of the growth stent frame 110 and more coverage in a center portion of the growth stent frame 110. The added flexibility and/or conformability can help ensure that the target location of the stent (or valve) frame 110 aptly opens narrowed vessels commonly found in patients with congenital heart disease and advantageously can conform to patient vasculature that is not narrowed.

In selected embodiments, the stent frame 100 can be provided with a first predetermined number of open cells 164A and/or a second predetermined number of closed cells 164B. The first predetermined number of open cells 164A can be increased to increase the conformability and/or flexibility of the stent frame 100. Additionally and/or alternatively, the first predetermined number of open cells 164A and/or the second predetermined number of closed cells 164B can be adjusted for selected applications of the growth stent 100. The first predetermined number of open cells 164A and/or the second predetermined number of closed cells 164B, for example, can be adjusted to fit various needs in vasculature. For example, a stent frame 110 that eliminates junctions 170 on every other cell 164 can be more conformable than a stent frame 110 with junctions 170 that are eliminated at every third cell 164. Having only one connected junction 170 on each row of struts 180 can help to provide a maximal amount of flexibility and/or conformability for the stent frame 100. Junctions 170 advantageously may be eliminated in any suitable pattern based upon a predetermined need in vasculature of a patient or other application of the growth stent 100.

Turning to Figs. 10A-B, an exemplary embodiment of the growth stent 100 is shown as comprising an elongated stent frame 110. The stent frame 110 of Fig. 10A includes a plurality of annular growth cell members 190. In selected embodiments, the growth cell members 190 can be provided in the manner shown and described in United States Patent No. 12,004,939, the entirety of which is incorporated by reference herein. The growth cell members 190, for example, can define a periphery 182 of the stent frame 110 and extend from a proximal end region 130 of the stent frame 110 to a distal end region 140 of the stent frame 110. As shown in Fig. 10A, the stent frame 110 can comprise a series (or sequence) of growth cell members 190 that span axially between the proximal and distal end regions 130, 140 and that can provide a radial periphery 182 for the stent frame 110. The sequence of growth cell members 190 thereby can define a central axial channel 120 of the elongated stent frame 110.

The growth cell members 190 as shown in Figs. 10A-B, for example, can include a proximal growth cell member $190_P$ that is disposed at the proximal end region 130 of the stent frame 110 and a distal growth cell member $190_D$ that is disposed at the distal end region 140 of the stent frame 110. In selected embodiments, the growth cell members 190 can include an intermediate growth cell member 190 that is disposed between the proximal growth cell member $190_P$ and the distal growth cell member $190_D$. The growth cell members 190, in other words, can include a plurality of intermediate growth cell members disposed between the proximal growth cell member $190_P$ and the distal growth cell member $190_D$.

As shown in Figs. 10A-B, the growth stent 100 can comprise a catheter-insertable and re-expandable flexible growth stent 100 for implantation in a patient. The growth stent 100 can comprise an elongated flexible stent frame 110 that includes first, second and third annular frame strut arrangements 190A, 190B, 190C each being disposed in axial alignment between a proximal end region 130 and a distal end region 140 of the flexible stent frame 110. The first annular frame strut arrangement 190A, for example, can comprise a first zigzag arrangement of first frame strut members 180S, 180T, 180W, 180X, 180L, 180M with first peaks 183PA, 183PB and first valleys 183VA, 183VB as illustrated in Fig. 10A. Fig. 10A also shows that the second annular frame strut arrangement 190B can comprise a second zigzag arrangement of second frame strut members 180U, 180V, 180Y, 180Z, 180N, 1800 with second peaks 186PC, 186PD and second valleys 186VC, 186VD.

The third annular frame strut 190C arrangement, in turn, can be disposed between the first and second annular frame strut arrangements 190A, 190B and can comprise a third zigzag arrangement of third frame strut members 181S, 181T, 181W, 181X, 181L, 181M. The third zigzag arrangement of third frame strut members 181S, 181T, 181W, 181X, 181L, 181M can include a third peak 184PA that can be coupled with a corresponding one of the first valleys 183VA to form a first strut junction 170A and/or a fourth peak 184PB that can be disposed adjacent to a corresponding one of the first valleys 183VB to define a first intermediate gap 192B. Additionally and/or alternatively, the third zigzag arrangement of third frame strut members 181S, 181T, 181W, 181X, 181L, 181M can include a third valley 184VC that can be coupled with a corresponding one of the second peaks 186PC to form a second strut junction 170C and/or a fourth valley 184VD that can be disposed adjacent to a corresponding one of the second peaks 186PD to define a second intermediate gap 192D. The first and second intermediate gaps 192B, 192D advantageously can help to enhance an axial flexibility of the flexible stent frame 110.

As illustrated in Fig. 10B, each of the third frame strut members 181S, 181T, 181W, 181X, 181L, 181M associated with the first strut junction 170A optionally can include proximal and distal end regions with respective cross-sections 1831XP, 1831XD that are less than a cross-section 1831XC of a central region of the third frame strut member 181S, 181T, 181W, 181X, 181L, 181M. Each of the first frame strut members 180S, 180T, 180W, 180X, 180L, 180M associated with the first strut junction 170A can include proximal and distal end regions with respective cross-sections 1811XP, 1811XD that are less than a cross-section 1811XC of a central region of the first frame strut member 180S, 180T, 180W, 180X, 180L, 180M in selected embodiments. Additionally and/or alternatively, each of the third frame strut members 181S, 181T, 181W, 181X, 181L, 181M associated with the second strut junction 170C can include proximal and distal end regions with respective cross-sections 1832XP, 1832XD that are less than a cross-section 1832XC of a central region of the third frame strut members 181S, 181T, 181W, 181X, 181L, 181M and/or each of the second frame strut members 180U, 180V, 180Y, 180Z, 180N, 1800 associated with the second strut junction 170C can include proximal and distal end regions with respective cross-sections 1822XP, 1822XD that are less than a cross-section 1822XC of a central region of the second frame strut member 180U, 180V, 180Y, 180Z, 180N, 1800.

In the manner discussed in more detail above, the flexible stent frame 110 can have an initial cross-section for facilitating implantation within a selected lumen of the patient. The flexible stent frame 110 can be configured to expand from the initial cross-section to a first stable expanded state for supporting the selected lumen at implantation. In the first stable expanded state, the flexible stent frame 110 can define an internal channel 120 with a first cross-section 188X. The flexible stent frame 110 can be further configured to subsequently expand from the first stable expanded state to a second stable expanded state as the patient and/or the selected lumen grow while maintaining the axial flexibility of the flexible stent frame 110. In the second stable expanded state, the flexible stent frame 110 can continue to support the selected lumen and can define the internal channel with a second cross-section 189X being larger than the first cross-section 188X.

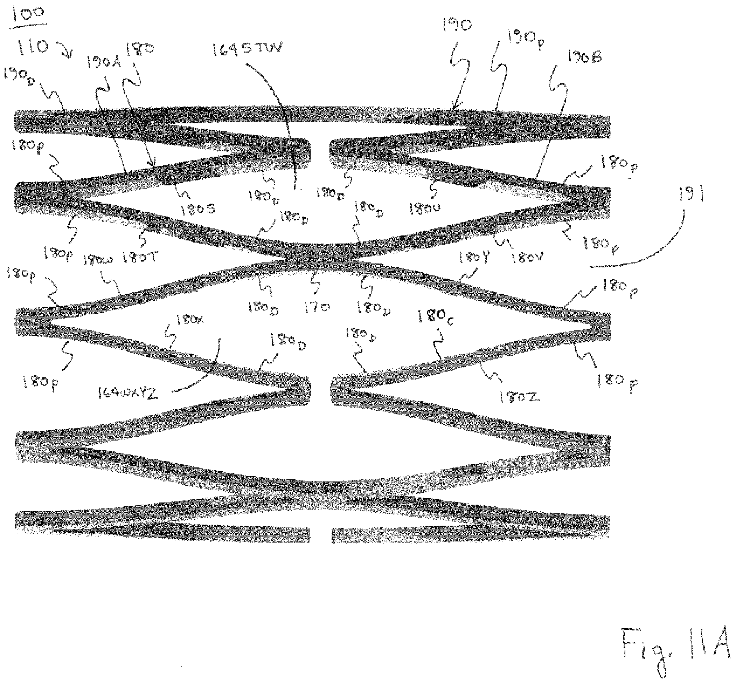

The growth cell members 190 of the stent frame 110 can have uniform structures and/or different structures, depending, for example, upon a predetermined application of the growth stent 100, an implant location of the growth stent 100 within a patient (not shown) and/or an anatomy of the patient. In selected embodiments, the growth cell members 190 can be formed from metal, metal alloys, polymer, biodegradable polymers, cloth, and/or a combination of multiple materials, without limitation. An exemplary growth cell member 190 is illustrated in Fig. 11A. Turning to Fig. 11A, the growth cell member 190 is shown as comprising a scaffolded growth cell member with a plurality of growth cell struts 180 that can be disposed in a ring (or annular) configuration for defining an internal growth cell opening 191 of the growth cell member 190.

The growth cell struts 180 can have predetermined cell strut widths and/or predetermined cell strut thicknesses and/or can define one or more frame cells 164. The cell strut widths and/or cell strut thicknesses can be increased to increase a radial strength of the growth cell member 190 and/or can be decreased to decrease the radial strength of the growth cell member 190. The frame cells 164 advantageously can enable the growth stent 100 to be crimped to an implantation state with a predetermined initial size, shape, diameter, cross-section or other dimension as well as to expand to a (stable) expanded state with a predetermined expanded size, shape, diameter, cross-section or other dimension.

In some embodiments, the stent frame 110 can comprise a balloon-expandable stent frame 110. The stent frame 110, in other embodiments, can comprise a self-expanding device frame using materials such as nitinol and other metal alloys. The stent frame 110 advantageously can allow for further expansion as the patient grows. Although shown and described as comprising uniform cell strut widths, uniform cell strut thicknesses and/or uniform frame cells 164 with reference to Fig. 11A for purposes of illustration only, the growth cell struts 180 can include cell strut widths, cell strut thicknesses and/or frame cells 164 that are uniform and/or different.

In selected embodiments, the growth cell struts 180 can be provided as one or more pairs of growth cell struts 180. Each pair of the growth cell struts 180 optionally can be paired with one or more other paired growth cell struts 180 of the growth cell member 190 as illustrated in Fig. 11A. The paired growth cell struts 180, for example, can include a first pair of growth cell struts 180S, 180T and a second pair of growth cell struts 180U, 180V. Each of the growth cell struts 180S, 180T, 180U, 180V can include a proximal end region $180_P$, and a central region $180c$ and a distal end region $180_D$.

With reference to the first pair of growth cell struts 180S, 180T, the proximal end region $180_P$ of the growth cell strut 180S can be coupled with the proximal end region $180_P$ of the growth cell strut 180T with the distal end regions $180_D$ of the growth cell struts 180S, 180T extending from the coupled proximal end regions $180_P$. The proximal end regions $180_P$ of the growth cell struts 180U, 180V of the second pair likewise can be coupled, and the distal end regions $180_D$ of the growth cell struts 180U, 180V can extend from the coupled proximal end regions $180_P$. As illustrated in Fig. 11A, the distal end region $180_D$ of the growth cell strut 180S of the first pair can be disposed adjacent to the distal end region $180_D$ of the growth cell strut 180U of the second pair; whereas, the distal end region $180_D$ of the growth cell strut 180T of the first pair can be disposed adjacent to the distal end region $180_D$ of the growth cell strut 180V of the second pair. The first and second pairs of growth cell struts 180S, 180T, 180U, 180V thereby can define a first frame cell 164STUV.

The distal end region $180_D$ of the growth cell strut 180S of the first pair optionally can be coupled with the distal end region $180_D$ of the growth cell strut 180U of the second pair, and/or the distal end region $180_D$ of the growth cell strut 180T of the first pair optionally can be coupled with the distal end region $180_D$ of the growth cell strut 180V of the second pair. In selected embodiments, the distal end region $180_D$ of the growth cell strut 180S can be separate from the distal end region $180_D$ of the growth cell strut 180U with the distal end regions $180_D$ as shown in Fig. 11A. The distal end region $180_D$ of the growth cell strut 180T optionally can be separate from the distal end region $180_D$ of the growth cell strut 180V with the distal end regions $180_D$.

Additionally and/or alternatively, the paired growth cell struts 180 can include a third pair of growth cell struts 180W, 180X and a fourth pair of growth cell struts 180Y, 180Z. Each of the growth cell struts 180W, 180X, 180Y, 180Z can include a proximal end region $180_P$, a central region 180c and a distal end region $180_D$. The proximal end region $180_P$ of the growth cell strut 180W can be coupled with the proximal end region $180_P$ of the growth cell strut 180X with the distal end regions $180_D$ of the growth cell struts 180W, 180X extending from the coupled proximal end regions $180_P$. The proximal end regions $180_P$ of the growth cell struts 180Y, 180Z of the fourth pair likewise can be coupled, and the distal end regions $180_D$ of the growth cell struts 180Y, 180Z extending from the coupled proximal end regions $180_P$. As shown in Fig. 11A, the distal end region $180_D$ of the growth cell strut 180W of the third pair can be disposed adjacent to the distal end region $180_D$ of the growth cell strut 180Y of the fourth pair; whereas, the distal end region $180_D$ of the growth cell strut 180X of the third pair can be disposed adjacent to the distal end region $180_D$ of the growth cell strut 180Z of the fourth pair. The third and fourth pairs of growth cell struts 180W, 180X, 180Y, 180Z thereby can define a second frame cell 164WXYZ.

The distal end region $180_D$ of the growth cell strut 180W of the third pair can be coupled with the distal end region $180_D$ of the growth cell strut 180Y of the fourth pair, and/or the distal end region $180_D$ of the growth cell strut 180X of the third pair can be coupled with the distal end region $180_D$ of the growth cell strut 180Z of the fourth pair. In selected embodiments, the distal end region $180_D$ of the growth cell strut 180W can be separate from the distal end region $180_D$ of the growth cell strut 180Y with the distal end regions $180_D$. The distal end region $180_D$ of the growth cell strut 180X optionally can be separate from the distal end region $180_D$ of the growth cell strut 180Z with the distal end regions $180_D$ as shown in Fig. 11A. Although the first, second, third and fourth pairs of growth cell struts 180 are shown and described with reference to Fig. 11A as being coupled in similar manners for purposes of illustration only, the growth cell struts 180 can be coupled in any suitable uniform and/or different manners.

The growth cell struts 180 can be coupled in any suitable manner to form one or more growth cell junctions 170 of the growth cell member 190. At least one of the growth cell junctions 170 optionally can comprise one or more arcuate members, such as arcuate members 174, 176, provided in the manner discussed in more detail above with reference to Figs. 4A-B. To form the growth cell junctions 170, the distal end regions $180_D$ of adjacent growth cell struts 180, for example, can be coupled. As illustrated in Fig. 11A, the distal end region $180_D$ of the growth cell strut 180T in the second pair of growth cell struts 180S, 180T and the distal end region $180_D$ of the growth cell strut 180W in the third pair of growth cell struts 180W, 180X can be coupled. The distal end region $180_D$ of the growth cell strut 180V in the second pair of growth cell struts 180U, 180V and the distal end region $180_D$ of the growth cell strut 180Y in the fourth pair of growth cell struts 180Y, 180Z likewise can be coupled.

In selected embodiments, the coupled distal end regions $180_D$ of the growth cell struts 180T, 180W can be coupled with the coupled distal end regions $180_D$ of the growth cell struts 180V, 180Y as shown in Fig. 11A. The coupling between the coupled distal end regions $180_D$ of the growth cell struts 180T, 180W and the coupled distal end regions $180_D$ of the growth cell struts 180V, 180Y can be provided as a growth cell junction 170 as discussed herein. Stated somewhat differently, the coupled distal end regions $180_D$ of the growth cell struts 180T, 180W and the coupled distal end regions $180_D$ of the growth cell struts 180V, 180Y can intersect to form the growth cell junction 170. The growth cell junction 170 advantageously can provide a mechanical (or physical) connection between the growth cell struts 180T, 180W and the growth cell struts 180V, 180Y to form the growth cell member 190. Preferably, the growth cell junction 170 can provide a flexible coupling between the coupled distal end regions $180_D$ of the growth cell struts 180T, 180W and the coupled distal end regions $180_D$ of the growth cell struts 180V, 180Y.

Each pair of coupled distal end regions $180_D$ for the growth cell struts 180 of the growth cell member 190 can be coupled via a respective growth cell junction 170. Alternatively, one or more pairs of the coupled distal end regions $180_D$ of the growth cell struts 180 can be separate (or not coupled). The separated pairs of the coupled distal end regions $180_D$ of the growth cell struts 180 can help enhance a flexibility of the growth cell member 190 and, thereby, the stent frame 110.

In the manner discussed in more detail above with reference to Equation 1, the proximal and distal end regions $180_P$, $180_D$ of the growth cell struts 180, in selected embodiments, can have an established diameter, cross-section and/or other dimension that is greater than an established diameter, cross-section and/or other dimension at the central region 180c of the growth cell struts 180 to isolate strain at the central region 180c. Alternatively, the established diameter, cross-section and/or other dimension of the end regions $180_P$, $180_D$ of the growth cell struts 180 can be less than the established diameter, cross-section and/or other dimension at the central portion 180c of the growth cell struts 180 for increasing the strain experienced by the junction 170 of the stent frame 110.

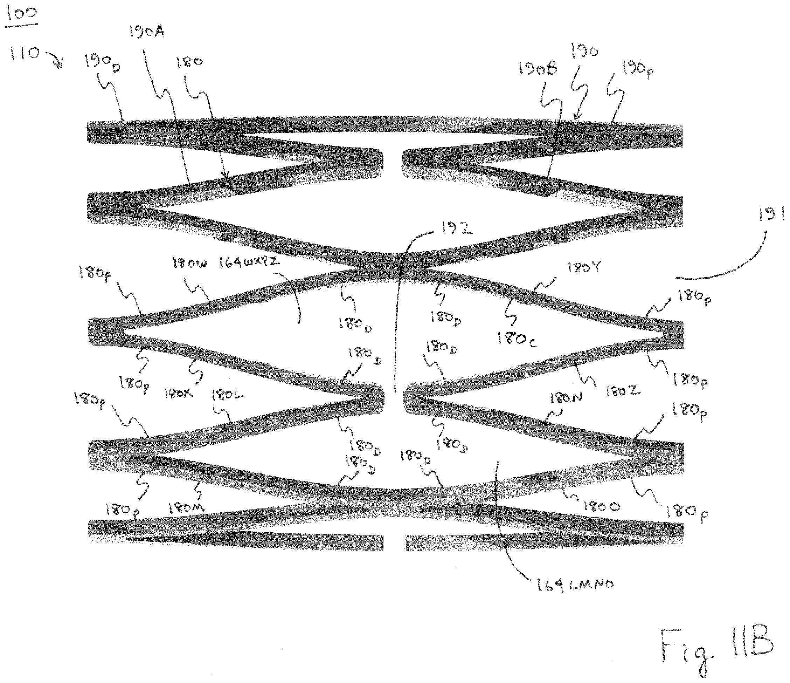

Turning to Fig. 11B, for example, the growth cell member 190 is shown as including a fifth pair of growth cell struts 180L, 180M and a sixth pair of growth cell struts 180N, 1800. The fifth pair of growth cell struts 180L, 180M and the sixth pair of growth cell struts 180N, 1800 can be provided in the manner discussed in more detail above with reference to the first pair of growth cell struts 180S, 180T and the second pair of growth cell struts 180U, 180V of Fig. 11A and can be configured to define a third frame cell 164LMNO. Coupled distal end regions $180_D$ of the growth cell struts 180L, 180X are shown as being separate from coupled distal end regions $180_D$ of the growth cell struts 180N, 180Z.

The coupled distal end regions $180_D$ of the growth cell struts 180L, 180X and the coupled distal end regions $180_D$ of the growth cell struts 180N, 180Z can form a gap, such as an apical gap 192, as illustrated in Fig. 11B. Stated somewhat differently, the growth cell member 190 can comprise a plurality of apical gaps 192 disposed between adjacent frame cells 164. The apical gaps 192 advantageously can enable the stent frame 110, and thus the growth stent 100, to bend and/or deflect. A flexibility of the stent frame 110 can be increased by increasing a size of at least one of the apical gaps 192 and/or can be decreased by decreasing the size of the apical gaps 192.

Additionally and/or alternatively, the proximal and distal end regions $180_P$, $180_D$ of the growth cell struts 180, in selected embodiments, can have an established diameter, cross-section and/or other dimension that is greater than an established diameter, cross-section and/or other dimension at the central region 180c of the growth cell struts 180 to isolate strain at the central region 180c in the manner discussed in more detail above with reference to Equation 1. The established diameter, cross-section and/or other dimension of the end regions $180_P$, $180_D$ of the growth cell struts 180 alternatively can be less than the established diameter, cross-section and/or other dimension at the central portion 180c of the growth cell struts 180 for increasing the strain experienced by the junction 170 of the stent frame 110.

In selected embodiments, the first pair of growth cell struts 180S, 180T, the third pair of growth cell struts 180W, 180X and the fifth pair of growth cell struts 180L, 180M of Figs. 11A-B can be coupled to form a first annular strut arrangement 190A of coupled growth cell struts 180; whereas, the second pair of growth cell struts 180U, 180V, the fourth pair of growth cell struts 180Y, 180Z and the sixth pair of growth cell struts 180N, 1800 of Figs. 11A-B can be coupled to form a second annular strut arrangement 190B of coupled growth cell struts 180. The growth cell struts 180 associated with the respective first and second annular strut arrangements 190A, 190B, in other words, can be coupled in a zigzag (or meandering) pattern to form a cylinder.

The proximal end regions $180_P$ of a pair of adjacent growth cell struts 180 can be coupled and the distal end regions $180_D$ of the pair of adjacent growth cell struts 180 can extend radially from the coupled proximal end regions $180_P$ to form a V shape, or half of a Z shape. Pairs of coupled growth cell struts 180 forming the V shape can be repeated around a circumference of the cylinder with the distal end regions $180_D$ of the pairs of coupled growth cell struts 180 being coupled to form a repeating pattern of V-shapes (or Z-shapes). The first and second annular strut arrangements 190A, 190B can be coupled via a growth cell junction 170 and/or can cooperate to define the internal growth cell opening 191 of the growth cell member 190. As discussed above with reference to Fig. 11A, the growth cell junction 170 can provide a coupling between the coupled distal end regions $180_D$ of the growth cell struts 180T, 180W and the coupled distal end regions $180_D$ of the growth cell struts 180V, 180Y. The growth cell junction 170 thereby can provide a flexible coupling between the first and second strut annular arrangements 190A, 190B.

In selected embodiments, the stent frame 110 can comprise a first, second and third annular frame strut arrangements (or annular growth cell members) 190A, 190B, 190C. The first annular frame strut arrangement 190A of the stent frame 110 can have a plurality of first peaks and first valleys, and the second annular frame strut arrangement 190B can have a plurality of second peaks and second valleys. The third annular frame strut arrangement 190C can be disposed between the first annular frame strut arrangement 190A and the second annular frame strut arrangement 190B and can include a third peak being coupled with a corresponding one of the first valleys to form a first strut junction 170 and a third valley being coupled with a corresponding one of the second peaks to form a second strut junction 170. In selected embodiments, the third peak can be directly coupled with the corresponding one of the first valleys to form the first strut junction 170, and/or the third valley can be directly coupled with the corresponding one of the second peaks to form the second strut junction 170.

In the manner discussed in more detail above with reference to the stent frame 110 of Figs. 10A-B, the growth cell members 190 can include a proximal growth cell member 190$_P$ that is disposed at the proximal end region 130 of the stent frame 110 and a distal growth cell member 190$_D$ that is disposed at the distal end region 140 of the stent frame 110. The proximal growth cell member 190$_P$ and the distal growth cell member 190$_D$ thereby can be disposed in axial alignment such that the internal growth cell openings 191 of the growth cell members 190 cooperate to define the internal channel 120 of the growth stent 100 and peripheries of the growth cell members 190 cooperate to form the periphery 182 (shown in Figs. 10A-B) of the growth stent frame 110. The proximal growth cell member 190$_P$ and the distal growth cell member 190$_D$ can be coupled in any suitable manner. The proximal end regions 180$_P$ of selected growth cell struts 180 of the proximal growth cell member 190$_P$, for example, can be directly and/or indirectly coupled with proximal end regions 180$_P$ of selected growth cell struts 180 of the distal growth cell member 190$_D$. Stated somewhat differently the proximal growth cell member 190$_P$ can be directly coupled with the distal growth cell member 190$_D$ as illustrated in Figs. 10A-B and/or the proximal growth cell member 190$_P$ and the distal growth cell member 190$_D$ can be indirectly coupled via at least one intermediate growth cell member 190 or other intermediate member of the stent frame 110.

The growth stent 100, additionally and/or alternatively, can include one or more junctions 170 with a non-uniform diameter, cross-section or other dimension. The diameter, cross-section or other dimension of the junctions 170, for example, can be varied, or reduced, to create additional strain on the junction 170. At least one area within the junction 170 optionally may be eliminated to reduce an amount of metal or other material comprising the stent frame 110. By removing material of the junction 170, strain may be further concentrated on the narrowed regions of the junction 170, and there is less junction material to deform, leading to increased displacement of the cells 164, more conformability to vasculature of the patient and general axial flexibility of the growth stent 100.

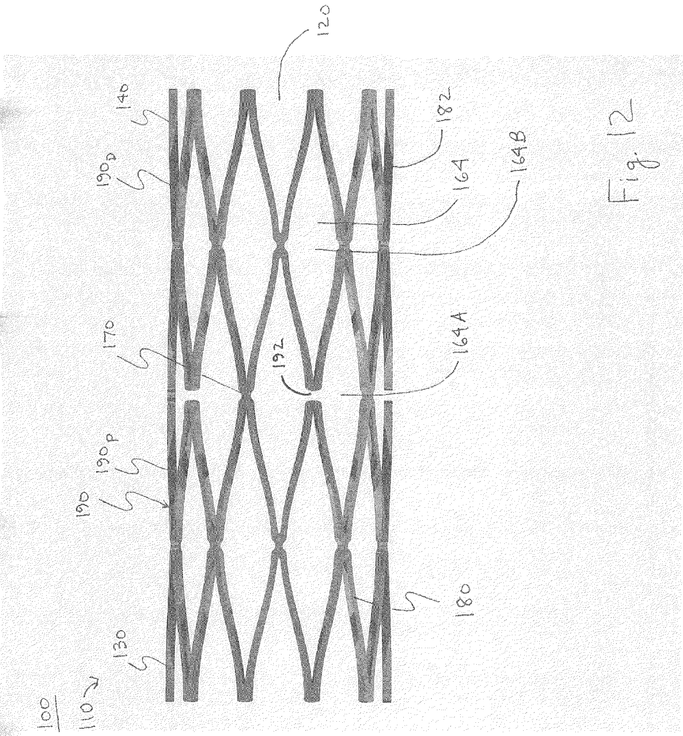

Turning to Fig. 12, for example, the growth stent 100 is shown as comprising an elongated stent frame 110. The stent frame 110 of Fig. 12 includes a plurality of annular growth cell members 190. In selected embodiments, the growth cell members 190 can be provided in the manner shown and described in United States Patent No. 12,004,939, the entirety of which is incorporated by reference herein. The growth cell members 190, for example, can define a periphery 182 of the stent frame 110 and extend from a proximal end region 130 of the stent frame 110 to a distal end region 140 of the stent frame 110. As shown in Fig. 12, the stent frame 110 can comprise a series (or sequence) of growth cell members 190 that span axially between the proximal and distal end regions 130, 140 and that can provide a radial periphery 182 for the stent frame 110. The sequence of growth cell members 190 thereby can define a central axial channel 120 of the elongated stent frame 110.

In selected embodiments, the stent frame 110 can comprise a first, second and third annular frame strut arrangements (or annular growth cell members) 190A, 190B, 190C. The first annular frame strut arrangement 190A of the stent frame 110 can have a plurality of first peaks and first valleys, and the second annular frame strut arrangement 190B can have a plurality of second peaks and second valleys. The third annular frame strut arrangement 190C can be disposed between the first annular frame strut arrangement 190A and the second annular frame strut arrangement 190B and can include a third peak being coupled with a corresponding one of the first valleys to form a first strut junction 170 and a third valley being coupled with a corresponding one of the second peaks to form a second strut junction 170.

The growth cell members 190 as shown in Fig. 12, for example, can include a proximal growth cell member 190$_P$ that is disposed at the proximal end region 130 of the stent frame 110 and a distal growth cell member 190$_D$ that is disposed at the distal end region 140 of the stent frame 110. In selected embodiments, the growth cell members 190 can include an intermediate growth cell member 190 that is disposed between the proximal growth cell member 190$_P$ and the distal growth cell member 190$_D$. The growth cell members 190, in other words, can include a plurality of intermediate growth cell members disposed between the proximal growth cell member 190$_P$ and the distal growth cell member 190$_D$.

The growth cell members 190 of the stent frame 110 can have uniform structures and/or different structures, depending, for example, upon a predetermined application of the growth stent 100, an implant location of the growth stent 100 within a patient (not shown) and/or an anatomy of the patient. In selected embodiments, the growth cell members 190 can be formed from metal, metal alloys, polymer, biodegradable polymers, cloth, and/or a combination of multiple materials, without limitation. As shown in Fig. 12, the stent frame 110 can comprise a plurality of struts 180. Selected struts 180 can intersect to form junctions 170. The junctions 170 can be provided in the manner discussed herein and, as illustrated in Fig. 12, can have a diameter, cross-section or other dimension that is varied, or reduced, to create additional strain on the junction 170. At least one junction 170 within the stent frame 110 optionally can be eliminated to define a gap, such as an apical gap 192, in the manner set forth in more detail herein.

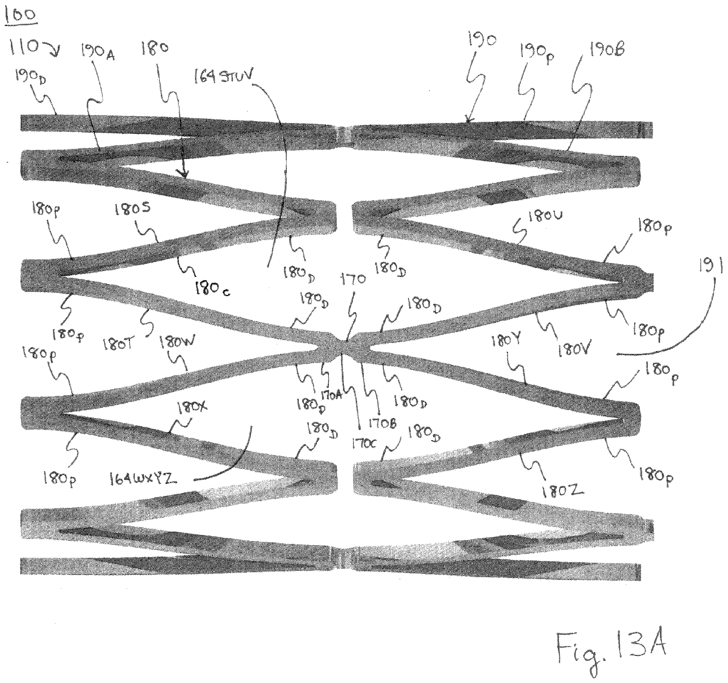

An exemplary growth cell member 190 is illustrated in Fig. 13A. Turning to Fig. 13A, the growth cell member 190 is shown as comprising a scaffolded growth cell member with a plurality of growth cell struts 180 that can be disposed in a ring (or annular) configuration for defining an internal growth cell opening 191 of the growth cell member 190. The growth cell struts 180 can have predetermined cell strut widths and/or predetermined cell strut thicknesses and/or can define one or more frame cells 164. The cell strut widths and/or cell strut thicknesses can be increased to increase a radial strength of the growth cell member 190 and/or can be decreased to decrease the radial strength of the growth cell member 190. The frame cells 164 advantageously can enable the growth stent 100 to be crimped to an implantation state with a predetermined initial size, shape, diameter, cross-section or other dimension as well as to expand to a (stable) expanded state with a predetermined expanded size, shape, diameter, cross-section or other dimension.

In some embodiments, the stent frame 110 can comprise a balloon-expandable stent frame 110. The stent frame 110, in other embodiments, can comprise a self-expanding device frame using materials such as nitinol and other metal alloys. The stent frame 110 advantageously can allow for further expansion as the patient grows. Although shown and described as comprising uniform cell strut widths, uniform cell strut thicknesses and/or uniform frame cells 164 with reference to Fig. 13A for purposes of illustration only, the growth cell struts 180 can include cell strut widths, cell strut thicknesses and/or frame cells 164 that are uniform and/or different.

In selected embodiments, the growth cell struts 180 can be provided as one or more pairs of growth cell struts 180. Each pair of the growth cell struts 180 optionally can be paired with one or more other paired growth cell struts 180 of the growth cell member 190 as illustrated in Fig. 13A. The paired growth cell struts 180, for example, can include a first pair of growth cell struts 180S, 180T and a second pair of growth cell struts 180U, 180V. Each of the growth cell struts 180S, 180T, 180U, 180V can include a proximal end region $180_P$, a central region 180c and a distal end region $180_D$.

With reference to the first pair of growth cell struts 180S, 180T, the proximal end region $180_P$ of the growth cell strut 180S can be coupled with the proximal end region $180_P$ of the growth cell strut 180T with the distal end regions $180_D$ of the growth cell struts 180S, 180T extending from the coupled proximal end regions $180_P$. The proximal end regions $180_P$ of the growth cell struts 180U, 180V of the second pair likewise can be coupled, and the distal end regions $180_D$ of the growth cell struts 180U, 180V can extend from the coupled proximal end regions $180_P$. As illustrated in Fig. 13A, the distal end region $180_D$ of the growth cell strut 180S of the first pair can be disposed adjacent to the distal end region $180_D$ of the growth cell strut 180U of the second pair; whereas, the distal end region $180_D$ of the growth cell strut 180T of the first pair can be disposed adjacent to the distal end region $180_D$ of the growth cell strut 180V of the second pair. The first and second pairs of growth cell struts 180S, 180T, 180U, 180V thereby can define a first frame cell 164STUV.

The distal end region $180_D$ of the growth cell strut 180S of the first pair optionally can be coupled with the distal end region $180_D$ of the growth cell strut 180U of the second pair, and/or the distal end region $180_D$ of the growth cell strut 180T of the first pair optionally can be coupled with the distal end region $180_D$ of the growth cell strut 180V of the second pair. In selected embodiments, the distal end region $180_D$ of the growth cell strut 180S can be separate from the distal end region $180_D$ of the growth cell strut 180U with the distal end regions $180_D$ as shown in Fig. 13A. The distal end region $180_D$ of the growth cell strut 180T optionally can be separate from the distal end region $180_D$ of the growth cell strut 180V with the distal end regions $180_D$.

Additionally and/or alternatively, the paired growth cell struts 180 can include a third pair of growth cell struts 180W, 180X and a fourth pair of growth cell struts 180Y, 180Z. Each of the growth cell struts 180W, 180X, 180Y, 180Z can include a proximal end region $180_P$, a central region 180c and a distal end region $180_D$. The proximal end region $180_P$ of the growth cell strut 180W can be coupled with the proximal end region $180_P$ of the growth cell strut 180X with the distal end regions $180_D$ of the growth cell struts 180W, 180X extending from the coupled proximal end regions $180_P$. The proximal end regions $180_P$ of the growth cell struts 180Y, 180Z of the fourth pair likewise can be coupled, and the distal end regions $180_D$ of the growth cell struts 180Y, 180Z extending from the coupled proximal end regions $180_P$. As shown in Fig. 13A, the distal end region $180_D$ of the growth cell strut 180W of the third pair can be disposed adjacent to the distal end region $180_D$ of the growth cell strut 180Y of the fourth pair; whereas, the distal end region $180_D$ of the growth cell strut 180X of the third pair can be disposed adjacent to the distal end region $180_D$ of the growth cell strut 180Z of the fourth pair. The third and fourth pairs of growth cell struts 180W, 180X, 180Y, 180Z thereby can define a second frame cell 164WXYZ.

The distal end region $180_D$ of the growth cell strut 180W of the third pair can be coupled with the distal end region $180_D$ of the growth cell strut 180Y of the fourth pair, and/or the distal end region $180_D$ of the growth cell strut 180X of the third pair can be coupled with the distal end region $180_D$ of the growth cell strut 180Z of the fourth pair. In selected embodiments, the distal end region $180_D$ of the growth cell strut 180W can be separate from the distal end region $180_D$ of the growth cell strut 180Y with the distal end regions $180_D$. The distal end region $180_D$ of the growth cell strut 180X optionally can be separate from the distal end region $180_D$ of the growth cell strut 180Z with the distal end regions $180_D$ as shown in Fig. 13A. Although the first, second, third and fourth pairs of growth cell struts 180 are shown and described with reference to Fig. 13A as being coupled in similar manners for purposes of illustration only, the growth cell struts 180 can be coupled in any suitable uniform and/or different manners.

The growth cell struts 180 can be coupled in any suitable manner to form one or more growth cell junctions 170 of the growth cell member 190. As illustrated in Fig.

13A, the junctions 170 can have a diameter, cross-section or other dimension that is varied, or reduced, to create additional strain on the junction 170. The junctions 170 optionally can be provided as flexible coupling members. The junction 170, for example, can comprise a central body 170C with first and second coupling regions 170A, 170B. The first coupling region 170A can be configured to couple with the coupled distal end regions 180D of the growth cell struts 180T, 180W; whereas, the second coupling region 1164B can be configured to couple with the coupled distal end regions 180D of the growth cell struts 180V, 180Y. At least one of the growth cell junctions 170 optionally can comprise one or more arcuate members, such as arcuate members 174, 176, provided in the manner discussed in more detail above with reference to Figs. 4A-B.

To form the growth cell junctions 170, the distal end regions $180_D$ of adjacent growth cell struts 180, for example, can be coupled. As illustrated in Fig. 13A, for example, the distal end region $180_D$ of the growth cell strut 180T in the second pair of growth cell struts 180S, 180T and the distal end region $180_D$ of the growth cell strut 180W in the third pair of growth cell struts 180W, 180X can be coupled. The distal end region $180_D$ of the growth cell strut 180V in the second pair of growth cell struts 180U, 180V and the distal end region $180_D$ of the growth cell strut 180Y in the fourth pair of growth cell struts 180Y, 180Z likewise can be coupled.

In selected embodiments, the coupled distal end regions $180_D$ of the growth cell struts 180T, 180W can be coupled with the coupled distal end regions $180_D$ of the growth cell struts 180V, 180Y as shown in Fig. 13A. The coupling between the coupled distal end regions $180_D$ of the growth cell struts 180T, 180W and the coupled distal end regions $180_D$ of the growth cell struts 180V, 180Y can be provided as a growth cell junction 170 as discussed herein. Stated somewhat differently, the coupled distal end regions $180_D$ of the growth cell struts 180T, 180W and the coupled distal end regions $180_D$ of the growth cell struts 180V, 180Y can intersect to form the growth cell junction 170. The growth cell junction 170 advantageously can provide a mechanical (or physical) connection between the growth cell struts 180T, 180W and the growth cell struts 180V, 180Y to form the growth cell member 190. Preferably, the growth cell junction 170 can provide a flexible coupling between the coupled distal end regions $180_D$ of the growth cell struts 180T, 180W and the coupled distal end regions $180_D$ of the growth cell struts 180V, 180Y.

Each pair of coupled distal end regions $180_D$ for the growth cell struts 180 of the growth cell member 190 can be coupled via a respective growth cell junction 170. Alternatively, one or more pairs of the coupled distal end regions $180_D$ of the growth cell struts 180 can be separate (or not coupled). The separated pairs of the coupled distal end regions $180_D$ of the growth cell struts 180 can help enhance a flexibility of the growth cell member 190 and, thereby, the stent frame 110.

In the manner discussed in more detail above with reference to Equation 1, the proximal and distal end regions $180_P$, $180_D$ of the growth cell struts 180, in selected embodiments, can have an established diameter, cross-section and/or other dimension that is greater than an established diameter, cross-section and/or other dimension at the central region 180c of the growth cell struts 180 to isolate strain at the central region 180c. Alternatively, the established diameter, cross-section and/or other dimension of the end regions $180_P$, $180_D$ of the growth cell struts 180 can be less than the established diameter, cross-section and/or other dimension at the central portion 180c of the growth cell struts 180 for increasing the strain experienced by the junction 170 of the stent frame 110.

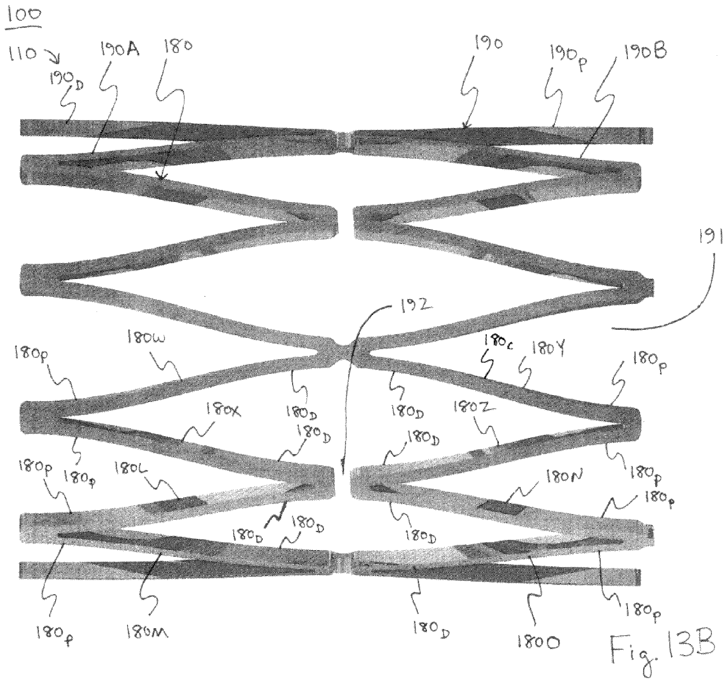

Turning to Fig. 13B, for example, the growth cell member 190 is shown as including a fifth pair of growth cell struts 180L, 180M and a sixth pair of growth cell struts 180N, 1800. The fifth pair of growth cell struts 180L, 180M and the sixth pair of growth cell struts 180N, 1800 can be provided in the manner discussed in more detail above with reference to the first pair of growth cell struts 180S, 180T and the second pair of growth cell struts 180U, 180V of Fig. 13A and can be configured to define a third frame cell 164LMNO. Coupled distal end regions $180_D$ of the growth cell struts 180L, 180X are shown as being separate from coupled distal end regions $180_D$ of the growth cell struts 180N, 180Z.

The coupled distal end regions $180_D$ of the growth cell struts 180L, 180X and the coupled distal end regions $180_D$ of the growth cell struts 180N, 180Z can form a gap, such as an apical gap 192, as illustrated in Fig. 13B. Stated somewhat differently, the growth cell member 190 can comprise a plurality of apical gaps 192 disposed between adjacent frame cells 164. The apical gaps 192 advantageously can enable the stent frame 110, and thus the growth stent 100, to bend and/or deflect. A flexibility of the stent frame 110 can be increased by increasing a size of at least one of the apical gaps 192 and/or can be decreased by decreasing the size of the apical gaps 192. Although shown and described as including the apical gap 192 with reference to Fig. 13B for purposes of illustration only, the stent frame 110 optionally can comprise a growth cell member 190 that includes no apical gaps 192 as shown in Fig. 12. The stent frame 110, in other words, can include a junction 170 at each intersection of growth cell struts 180 in selected embodiments.

Additionally and/or alternatively, the proximal and distal end regions $180_P$, $180_D$ of the growth cell struts 180, in selected embodiments, can have an established diameter, cross-section and/or other dimension that is greater than an established diameter, cross-section and/or other dimension at the central region 180c of the growth cell struts 180 to isolate strain at the central region 180c in the manner discussed in more detail above with reference to Equation 1. The established diameter, cross-section and/or other dimension of the end regions 180$_P$, 180$_D$ of the growth cell struts 180 alternatively can be less than the established diameter, cross-section and/or other dimension at the central portion 180c of the growth cell struts 180 for increasing the strain experienced by the junction 170 of the stent frame 110.

In selected embodiments, the first pair of growth cell struts 180S, 180T, the third pair of growth cell struts 180W, 180X and the fifth pair of growth cell struts 180L, 180M of Figs. 13A-B can be coupled to form a first annular strut arrangement 190A of coupled growth cell struts 180; whereas, the second pair of growth cell struts 180U, 180V, the fourth pair of growth cell struts 180Y, 180Z and the sixth pair of growth cell struts 180N, 1800 of Figs. 13A-B can be coupled to form a second annular strut arrangement 190B of coupled growth cell struts 180. The growth cell struts 180 associated with the respective first and second annular strut arrangements 190A, 190B, in other words, can be coupled in a zigzag (or meandering) pattern to form a cylinder.

The proximal end regions 180$_P$ of a pair of adjacent growth cell struts 180 can be coupled and the distal end regions 180$_D$ of the pair of adjacent growth cell struts 180 can extend radially from the coupled proximal end regions 180$_P$ to form a V shape, or half of a Z shape. Pairs of coupled growth cell struts 180 forming the V shape can be repeated around a circumference of the cylinder with the distal end regions 180$_D$ of the pairs of coupled growth cell struts 180 being coupled to form a repeating pattern of V-shapes (or Z-shapes). The first and second annular strut arrangements 190A, 190B can be coupled via a growth cell junction 170 and/or can cooperate to define the internal growth cell opening 191 of the growth cell member 190. As discussed above with reference to Fig. 13A, the growth cell junction 170 can provide a coupling between the coupled distal end regions 180$_D$ of the growth cell struts 180T, 180W and the coupled distal end regions 180$_D$ of the growth cell struts 180V, 180Y. The growth cell junction 170 thereby can provide a flexible coupling between the first and second strut annular arrangements 190A, 190B.

In the manner discussed in more detail above with reference to the stent frame 110 of Fig. 12, the growth cell members 190 can include a proximal growth cell member 190$_P$ that is disposed at the proximal end region 130 of the stent frame 110 and a distal growth cell member 190$_D$ that is disposed at the distal end region 140 of the stent frame 110. The proximal growth cell member 190$_P$ and the distal growth cell member 190$_D$ thereby can be disposed in axial alignment such that the internal growth cell openings 191 of the growth cell members 190 cooperate to define the internal channel 120 of the growth stent 100 and peripheries of the growth cell members 190 cooperate to form the periphery 182 (shown in Fig. 12) of the growth stent frame 110. The proximal growth cell member 190$_P$ and the distal growth cell member 190$_D$ can be coupled in any suitable manner. The proximal end regions 180$_P$ of selected growth cell struts 180 of the proximal growth cell member 190$_P$, for example, can be directly and/or indirectly coupled with proximal end regions 180$_P$ of selected growth cell struts 180 of the distal growth cell member 190$_D$. Stated somewhat differently the proximal growth cell member 190$_P$ can be directly coupled with the distal growth cell member 190$_D$ as illustrated in Fig. 12 and/or the proximal growth cell member 190$_P$ and the distal growth cell member 190$_D$ can be indirectly coupled via at least one intermediate growth cell member 190 or other intermediate member of the stent frame 110.

Certain rows of cells 164 can be more flexible and/or conformable by decreasing the amount of material of selected junctions 170 relative to other junctions 170. As an example, a central row of cells 164 from an axial perspective of the stent frame 110 can include junctions 170 with less removed material than junctions 170 associated with outside rows of cells 164, creating more flexibility and/or conformability at the outer rows of cells 164. The additional flexibility and/or conformability advantageously can help ensure that the stent (or valve) frame 110 aptly opens narrowed vessels commonly found in patients with congenital heart disease and/or can conform to vasculature within the patient that is not narrowed.

In selected embodiments, the stent frame 110 can include one or more instances of removed junctions 170 and/or reduced-dimension junctions 170 to create varying levels of flexibility and apical engagement. Removal and reduction of junctions 170 in predetermined patterns can help allow for control of flexibility and/or conformability. Additionally and/or alternatively, the stent frame 110 can be provided with a preselected combination of open-cell configurations and/or junctions 170 with one or more arcuate members, such as arcuate members 174, 176, provided in the manner discussed in more detail above with reference to Figs. 4A-B. The growth stent 100 advantageously can provide specific levels of conformability and/or flexibility with all manner of joint and junction combinations while enabling a small crimp diameter for deliverability in patients with congenital heart disease. Some embodiments of the stent frame 110 may lead to increasingly open-cell designs with expansions. For example, varying the connections of cells 164 can allow for star-shaped or other expansions such that the stent frame 110 can be crimped with cells 164 in a row but expanded in a pattern only limited by a length of the struts 180.

In the manner discussed in more detail above with reference to Figs. 1A-B and Figs. 2A-D, the growth stent 100 of Figs. 4A-B advantageously can be expanded to cover the wide range of anatomical diameters necessary to treat narrowed lesions across an entire lifetime of the patient. Stated somewhat differently, the predetermined number of expanded states supported by the growth stent 100 can configure the growth stent 100 to provide a wide range of anatomical diameters. The growth stent 100, for example, can be crimped on the delivery catheter 210 at small enough sizes for a neonatal patient, such as considerably less than an 8 French crimped growth stent 100, with an ability to be expanded later to teen and/or adult sizes of greater than 18mm in diameter. In selected embodiments, the growth stent 100 can be expanded to a diameter, cross-section or other dimension of 24mm and beyond.

Figs. 5A-D illustrate selected embodiments of a growth stent frame at various cycles throughout a patient's life, as the diameter of a vessel (or lumen) 300 (shown in Figs. 8A-D) of the patient grows. In this specific example, the growth stent frame 110 can maintain strength at the various diameters. Turning to Fig. 5A, the growth stent 100 is shown in the initial state for implantation. The stent body 110, in the initial state, can have a predetermined initial size, shape, diameter, cross-section or other dimension of, for example, 2mm for facilitating insertion of the growth stent 100 into the lumen 300. Throughout an entire growth cycle of the stent frame 110, a radial strength of the stent frame 110 can be maintained. In selected embodiments, as the stent frame 110 reaches increased diameters, a radial strength of the stent frame 110 can be increased.

As the angles of the elongated struts 180 in relation to connected elongated struts 180 become increasingly obtuse with increasing diameter, the force require to crimp the growth stent 100 can increase. Elongated struts 180 that are connected by a junction 170 or other vertex or joint, in other words, can define an angle that can become increasingly obtuse with increasing diameter and thus require an increased crimping force. When the growth stent 100 is expanded, for example, an angle defined between two connected struts 180 can become more obtuse, and the connected struts 180 can be harder to crimp because the increasingly obtuse angle. As diamonds or cells 164 widen, a radial force require to crimp the growth stent 100 can increase because the angle at which the struts 100 can be pushed is very different. Stated somewhat differently, crimping or otherwise closing connected struts 10 that define a tight V-shape can be easier than crimping or otherwise closing connected struts 10 that define a wider V-shape if a (horizontal) force is applied to the V-shape of the growth stent 100.

When the growth stent 100 is in an expanded state, the stent body 110 can define the internal channel 120 with any suitable predetermined diameter, cross-section or other dimension D. The stent body 110, for example, can define the internal channel 120 with a first predetermined diameter, cross-section or other dimension D of 8mm as shown in Fig. 5B, a second predetermined diameter, cross-section or other dimension D of 15mm as shown in Fig. 5C and/or a third predetermined diameter, cross-section or other dimension D of 20mm or more as shown in Fig. 5D, without limitation. The growth stent 100 thereby can be provided to hold open a narrow vessel 300 (shown in Figs. 8A-D) and allow for normal flow of blood or other bodily fluids.

As an example, the growth stent 100 can be implanted in a vessel (or lumen) 300 (shown in Figs. 8A-D) of a pediatric patient, a congenital patient, a cardiac patient or other suitable patient through expansion to a diameter of less than 10mm and have proper strength to be expanded over the course of a patient's lifetime to significantly greater than 18mm. The exemplary growth stent 100 can be tracked through the vessel 300 of the infantile patient via a delivery catheter 210 of a catheter delivery system 200 (collectively shown in Figs. 7A-B) that is considerably less than 8 French in diameter and, in some embodiments, less than 5 French in diameter and beyond.

Turning to Figs. 6A-B, growth stent geometry, including frame strut width 160, frame thickness 162, number of cells 164, a strut length of the strut 180 (shown in Figs. 10A-B), and/or cell angulation 166, can contribute to the ability to maintain strength over the wide range of deployments and expansions. Frame strut width 160, the strut length and the frame thickness 162 can be employed to determine the radial force of the growth stent frame 110 with the number of cells 164 magnifying the impact of these characteristics. In one embodiment, the growth stent frame 110 can be optimized to lessen the impact of foreshortening of the growth stent 100. Foreshortening is the length shortening that can occur as the growth stent 100 is expanded from the crimped state into the selected expanded state. The cell angulation 166 and length of the growth stent cells 164 can be optimized to lessen the amount of growth stent foreshortening in order to more predictably deploy the growth stent 100 through expansion, such as balloon expansion.

The width of the strut junctions 170 can contribute to an ability of the growth stent 100 to be crimped small enough to track through infantile blood vessels 300. Keeping the strut junction width 168 small to allow for the number of cells 164 in the growth stent 100 to be crimped smaller can help facilitate delivery. The junction width 168 preferably is large enough to expand over a large range for later expansion without significant frame stress and strain, leading to a small range in order to optimize the growth stent junction width 168 for this purpose. Growth stent frame material can enable these properties, allowing for strength across the wide range of operation despite the thin-walled nature of the growth stent 100 to allow the growth stent 100 to be crimped to small enough sizes to be delivered into neonatal patients.

The growth stent frame 110 may be optimized to allow for a thicker wall thickness 162 from the initial tubing utilized for the metal structure. Limiting junction width 168 to a minimum with relation to the strut width 160 can allow for an increase in wall thickness 162 of the frame 110, which can prevent the growth stent 100 from having a spring-like reaction when radial force is applied. For example, a junction width 168 of a junction 170 can contain a predetermined amount of separation between the struts 180 (shown in Figs. 10A-B) associated with the junction 170. The predetermined amount of separation can affect an ability of the stent frame 110 to be crimped and/or a general radial strength of the stent frame 110. Increasing the junction width 168 can permit an increased amount of material to form the junction 170 and thereby can require an increased amount of strain for plastic deformation, increasing the radial strength of the growth stent 100 and decreasing a recoil of the stent frame 110.

Additionally and/or alternatively, increasing the strut width 160 can produce one or more similar effects on the stent frame 110. Increasing the junction width 168 (or increasing the strut width 160 that can bear upon the junction width 168), however, can increase a minimum diameter of crimp because more material can be disposed across a circumference of the stent frame 110. If the junction width 168 is limited and an increase in wall thickness is obtained, a crush resistance of the stent frame 110 can be increased, lessening an effect of recoil from the vessel 300, increasing the radial force of the growth stent 100 across all diameters necessary for expansion and/or providing a rigid structure to hold the vessel 300 open.

Because of the strength needed for this patient population and a desire for the frame to be crimped to exceedingly small diameters, specific formulas exist for calculating strut width 160, wall thickness 162, junction size, and number of cells 164 that create an optimized version that allows for re-expansion over the large range of use for the growth stent 100. One exemplary formula for optimizing the growth stent 100 is set forth in Equation 1. According to Equation 1, the smallest crimp diameter of the growth stent 100 can be represented as:

$$\frac{(SW + J) * N}{\pi} + (2 * ST) \qquad \text{(Equation 1)}$$

wherein SW represents a strut width 160, J represents a junction width 168, N represents a number of cells 164, and ST represents a strut thickness from the wall thickness 162 of the initial tubing. Equation 1 allows for certain parameters to be set based upon the recoil strength necessary to maintain proper strength across the desired range of use, by increasing the strut thickness 162 and maintaining the ability to be crimped to desired small diameters. By minimizing the junction as only slightly larger than the strut width 160 of the growth stent 100, the smallest crimp diameter may be calculated as a function of strut width 160 and junction width 168 multiplied by the number of cells 164 in accordance with Equation 1.

Applying Equation 1 to determine a desired crimp size for the growth stent 100, the stent frame 110 preferable can maintain an adequate length of the elongated struts 180 (shown in Figs. 10A-B) within the patterns of cells 164 to accomplish the desired expansion of the diameter, cross-section or other dimension D (shown in Figs. 2B-D) of the stent frame 110. Increasing a length of the struts 180 advantageously can enable the maximum attainable diameter, cross-section or other dimension D of the stent frame 110 to increase, sometimes at a cost of radial force and/or recoil. In selected embodiments, a minimum crimp and/or a maximum expansion diameter of the stent frame 110 can be chosen for determining a length of the elongated struts 180. The strut width 160 then can be maximized and/or junction width 168 can be optimized for greatest radial strength of the stent frame 110. Increasing the number of cells 164 advantageously can permit decreased elongated strut length to maintain the same ultimate expansion diameters, but the strut width 160 and/or the junction width 168 preferably can be decreased to help maintain a minimum crimp diameter.

In some embodiments of the growth stent 100, a thickness 162 of the body (or frame) wall 112 can be minimized to prevent thrombus and/or stenosis of the vessel 300. Increasing the thickness 162 of the frame wall 112 can lessen the diameter of the vessel 300 after expansion as the frame wall 112 can provide blockage of the vessel 300. Additionally and/or alternatively, an increase in the thickness 162 of the frame wall 112 can provide more material for thrombus attachment. More material in the stent frame 110 can allow for more endothelization, allowing for cells 164 to attach to the foreign body. In selected embodiments, decreasing the wall thickness 162 of the frame wall 112 can increase a flexibility and/or a conformability to the vessel wall of the patient.

In selected embodiments, one or more of the strut width 160, the strut thickness 162, the strut length and/or other factors of the stent frame 110 can be varied and established at respective predetermined values. The elongated struts 180, for example, can have proximal and distal end regions $180_P$, $180_D$ (shown in Figs. 11A-B and 13A-B) with an established diameter, cross-section and/or other dimension that is greater than an established diameter, cross-section and/or other dimension at a central region (or portion) 180c (shown in Figs. 11A-B and 13A-B) of the strut 180 to isolate strain at the central portion of the strut 180. Alternatively, the established diameter, cross-section and/or other dimension of the end regions $180_P$, $180_D$ can be less than the established diameter, cross-section and/or other dimension at the central portion 180c of the strut 180 for increasing the strain experienced by the junction 170 of the stent frame 110.

In an optimized embodiment of the growth stent 100, the factors of strength and thickness 162 can be calculated to produce the best results for a growing patient. Utilizing vessel strength research, the growth stent frame may be optimized to allow for adequate strength across the varied range of operation utilizing one or more of the above factors. The growth stent 100 may have specific parameter ranges that are unique to this application, differentiating it from conventional stent frames. When attaching a resulting stent radial force and crush force as an output, for example, Equation 1 may be optimized for strength, allowing for an optimized stent frame 110 for growth over a specific range.

Equation 1 assumes that a measured length of the growth stent frame strut is constant and determined because changing the strut length can impact the output strength. In one optimized embodiment of the growth stent 100, the strut length can be determined to ensure that the stent frame 110 does not experience high levels of stress and strain that may lead to frame complications such as deformation and/or fracture. Increasing the length of the strut itself can increase the diameter of expansion but can decrease the resulting radial force. Therefore, an optimized embodiment of the growth stent 100 may contain the smallest strut length that allows for the stress and strain on the stent frame 110 to be acceptable across the intended diameter range of the growth stent 100.

Once the smallest strut length for the growth stent 100 is determined, Equation 1 may be utilized to optimize the frame 110. While two or more factors, or, in some cases, all factors may be changed in concert, certain parameter changes can show a pattern with regard to output radial strength of the frame 110, allowing for varied embodiments of the same stent structure to serve the purpose of a growth stent 100. In some embodiments of the growth stent 100, these parameters can be optimized for a small enough minimum crimp to not cause harm to vessels 300 of neonatal patients while providing adequate strength at large adult vessel sizes unlike commercially-available stents that are optimized for very specific adult-size diameter ranges.

The growth stent 100 preferably possesses adequate radial strength to keep the vessel 300 open at a specified diameter, such as by using the stent expansion system 260 (shown in Fig. 7A), such as a balloon, to expand the growth stent 100 to the specific diameter and the growth stent frame 110 containing enough radial strength to maintain the specific diameter without support from the balloon. However, radial strength and other properties of the growth stent 100 may affect the fatigue potential of the growth stent frame 110. Fatigue can result from continuous and repetitive motion creating stress and strain on the frame 110.

In some embodiments, one or more of the properties of the stent frame 110 can be optimized to ensure that unacceptable fatigue does not occur over the lifetime of the growth stent 100. This can be done in a variety of ways. Exemplary manners for avoiding unacceptable fatigue can include increasing the radial strength of the growth stent 100 to limit cyclical compression and expansion in the vessel 300 and/or increasing the length of the struts themselves to create more relaxed cell structures that are less susceptible to fatigue. The level of ductility in the metal or other material chosen for the stent frame 110 may also factor into the level of strain. Excess fatigue and strain on the growth stent frame 110 may lead to deformation and/or potential fracture of the metal or other material forming the growth stent 100. The stent frame 110 advantageously can be designed, using suitable materials and/or cell structures, to help maintain integrity of the growth stent 110 over the course of a lifetime of fatigue.

In some embodiments of the growth stent 100, the stent frame 110 can be cut from a tube using a laser cutting machine (not shown). The laser cutting machine can finely cut through the wall thickness 162 of the tubing to create the cell structure. After cutting, the unwanted material can be stripped and/or removed from between the cell struts that have been cut. In many cases, the laser cutting machine can leave rough edges on the growth stent frame 110 and/or the metal frame, which preferably are sanded on the inner diameter to ensure no burrs remain.

The radial strength, minimum crimp, growth range and other features of the growth stent 100 advantageously can be adjusted by utilizing one or more optimized materials. Utilizing complex metals or metal alloys, such as stainless steel, cobalt chromium and/or nitinol, for example, can have differing effects on the structure and operation of the growth stent 100. In an exemplary embodiment, cobalt chromium can be utilized to minimize the strut width and wall thickness of the growth stent 100 because cobalt chromium is stronger than other metals or metal alloys, such as platinum-iridium and/or stainless steel.

Additionally and/or alternatively, manufacturing processes have improved over the last few decades. Current manufacturing processes can enable the stent frame 110 and other metal tubes to be cut with finer and finer lasers, enabling smaller junction widths necessary to accomplish the minimum crimp desired for applications of the growth stent 100. When utilizing cobalt chromium or other complex metals or metal alloys, the strut width 160 may be less than 0.22mm and, in some cases, less than 0.2mm with a suitable number of cells 164. To help realize the minimum crimp, these complex metals can allow for wall thicknesses 162 that are less than 0.3mm and, in some cases, less than 0.25mm while still providing the necessary strength for the growth stent 100. Fine lasers and complex materials like cobalt chromium advantageously can enable the junction width 168 to be as close to the minimum strut widths 160 set forth herein. Various cobalt chromium, nitinol, stainless steel and/or other metal alloys optionally may be utilized to provide the growth stent 100.

Additionally and/or alternatively, some embodiments of the growth stent frame 110 can be electropolished to remove the outer layer of metal on the frame 110, smoothing the edges and finishing the frame 110 for implantation. Electropolishing in many instances uses electrical current through a liquid bath to evenly distribute the removal of material from the outer layers of the metal on the frame 110, often leaving the growth stent 100 with the appearance of shine from the polish. Some embodiments, such as frames utilizing nitinol or other shape-changing materials, may be shape-set to desired shapes using heat and quenching.

The diameter of the tubing utilized as the base of the stent frame 110 may affect the strength of the frame 110. Larger diameter tubes are less trapezoidal if the wall thickness 162 of the tubing is maintained. While the stent frame 110 may be adjusted to be cut at any diameter of tube as long as the wall thickness 162 remains accurate, trapezoidal struts that occur from smaller tubes may lead to less radial strength over the desired range as the strut is made with less material. In an optimized version of the growth stent 100, the frame 110 can be cut from a specific tubing thickness 162 and/or diameter to ensure proper strength across the entire range of use.

In selected embodiments, the growth stent 100 may include a bare metal frame 110 and/or have a blood-impermeable covering on a segment of the frame 110. The blood-impermeable covering allows for vessel ingrowth and seals the vessel 300 from blood leaking through the growth stent 100. The covering may be of cloth material such as polyethylene terephthalate (PET) and/or a fluoropolymer, such as some polytetrafluoroethylenes. The covering seals and performs across the range of diameters of the growth stent frame 110 and is able to be expanded with the frame 110 over the lifetime of patient growth. In one embodiment, the blood-impermeable covering can be attached to each diamond-shaped cell of the growth stent 100, form-fitting to each cell. The cloth in this embodiment may be sewed or tacked onto the frame 110 using a suture or other suitable tacking method.

In one exemplary embodiment, the blood-impermeable covering can be attached at the proximal and distal end regions 130, 140 (shown in Figs. 1A-B) of the growth stent frame 110 and/or at one or more other predetermined locations along the growth stent frame 110 to allow for proper expansion. The covering may be attached in any suitable manner, such as through sewing and/or tacking in the manner set forth herein and/or through selective welds to ensure fixture to the frame 110. Additionally and/or alternatively, the growth stent frame 110 may be dipped in a polymer that forms a flexible webbing between the cells 164 of the frame 110 to provide a blood-impermeable covering. In this embodiment, the frame 110 can be dipped in a liquid polymer and slowly removed, allowing the polymer to dry and create the flexible covering after removal. The polymer may be cured in air and/or in an oven for desired material properties.

One or both end regions 130, 140 of the growth stent 100 may be flared outwardly relative to a longitudinal axis of the growth stent 100 in some embodiments. The flared end regions 130, 140 advantageously can help secure the growth stent 100 into place in the wall of the artery or other lumen. The flare may be implemented in any suitable manner. For example, the flare may be implemented by outwardly curving the tips of a selected end region 130, 140 using material properties and/or shape-setting. Another embodiment can use expansion from a balloon or other expandable member (not shown) with an outward curve shape. Additionally and/or alternatively, one or both end regions 130, 140 may be flared inwardly to prevent aneurysm using similar techniques.

Aneurysms may be similarly prevented through dulling one or both end regions 130, 140 of the growth stent 100. Dulled end regions 130, 140 may be created in the growth stent 100, in some embodiments, by attaching circular eyelets and/or ends of various sizes to lessen the sharpness of the growth stent frame 110. For example, the end regions 130, 140 may be circular, hooked, or have any other non-invasive shape. Another embodiment of the growth stent 100 can use features on the growth stent 100 to engage with the anatomy and keep the growth stent 100 in place. In this embodiment, the growth stent 100 can contain sharper edges cut into the growth stent frame 110 and/or may utilize a type of barb for fixturing, whether through the growth stent frame 110 or the addition of suture knots to provide fixation in the vessel 300.

In another embodiment of the stent frame 110, one or both of the end regions 130, 140 may have material removed with relation to the rest of the stent frame 110. The material can be removed, for example, from the strut width 160 of the stent frame 110, from the strut thickness 162 of the growth stent 100 or from both. Removing the material allows for the thinned end regions 130, 140 of the growth stent 100 to be more flexible than the thicker stent body 110, allowing for less blood vessel penetration and a potential to be less invasive in the patient's body. Flexibility may be utilized in this embodiment of the frame 110 to prevent aneurysms and vessel irritation. If the embodiment of the growth stent 100 is balloon dilatable, for example, the thinned end regions 130, 140 can cause less resistance to the balloon and may be utilized as tool to prevent movement of the growth stent 100 during deployment utilizing the balloon.

The end regions 130, 140 of the growth stent 100 can engage into the surrounding vessel 300 or other anatomy in the narrowed lesion using radial force. Flares at the end regions 130, 140 advantageously can be applied for securing the growth stent 100 to the surrounding anatomy. The flares serve as a stop, which can secure the growth stent 100 in place. When an axial force is applied to the growth stent 100, the flares can push into the surrounding anatomy to resist migration of the growth stent 100.

The flares and growth stent expansion may be done naturally through expanding properties of the growth stent 100, such as material choice, and/or manually through the use of a pressured balloon or other stent expansion system 260 to expand the frame 110 to a larger diameter than the vessel 300, stretching the walls of the vessel 300 and anchoring the growth stent 100 within the vessel 300 using radial strength. As the patient grows, the diameter of the growth stent 100 can increase through material properties and self-expansion and/or through a later balloon dilation to increase the diameter and ensure the growth stent 100 maintains fixation through over-expansion and resulting radial force of the frame 110.

The growth stent 100 may be made of one or more of a variety of conventional materials for balloon-expanding stents or, in alternative embodiments, for self-expandable growth stents. As non-limiting examples, the growth stent 100 may be made of any appropriate material, such as a metal or metal alloy, including stainless steel, cobalt chromium, nitinol, or Elgiloy, or a polymer, for example. For self-expanding embodiments, the growth stent 100 can be made of a shape memory material such as, for example, nitinol.

The growth stent 100 advantageously can be configured for delivery into narrowed vessels 300 with an ability to be expanded to a selected expanded state, such as an adult size, and maintain adequate strength for vessel openings over the entire range of necessary expansion. In selected embodiments, a transvascular technique can be used to introduce and implant the growth stent 100 in neonates and other young patients using a catheter delivery system 200 as shown in Figs. 7A-B. Turning to Fig. 7A, the exemplary catheter delivery system 200 can comprise a flexible delivery catheter 210 and advantageously can introduce and implant the growth stent 100 in a manner that is less invasive than open heart surgery. The delivery catheter 210 can be trackable to a heart (or other implantation site 300 (shown in Figs. 8A-D)) and surrounding vasculature through a vessel 300 in the groin or another connecting vessel 300.

An end portion 220 of the delivery catheter 210 is shown as including an inner shaft 240 and an outer shaft 250. One or more of the shafts 240, 250 can be made of a flexible plastic, such as Pebax or other Nylons and contain open lumens. The delivery catheter 210 preferably comprises a flexible catheter. The flexibility can allow for proper pushability through human vascular to allow the growth stent 100 to be delivered to a proper location. The plastics used for the delivery catheter 210 can be biocompatible as to not cause harm to the patient.

In selected embodiments, the growth stent 100 can be mounted in the initial (or crimped) state on the end portion 220 of the delivery catheter 210. In other words, the growth stent 100 in the initial state can receive the inner shaft 240 via the internal channel 120 (shown in Figs. 1A-B). The growth stent 100 thereby can be disposed between the inner shaft 240 and the outer shaft 250 of the delivery catheter 210. The end portion 220 of the delivery catheter 210 likewise can include an optional stent expansion system 260, such as a balloon, for expanding the growth stent 100 from the initial state to a predetermined expanded state. In selected embodiments, the stent expansion system 260 can be at least partially disposed within the internal channel 120 to facilitate subsequent expansion of the growth stent 100.

Once the growth stent 100 is properly positioned on the end portion 220 of the delivery catheter 210, the catheter delivery system 200 can be provided in a closed configuration as illustrated in Fig. 7B. Stated somewhat differently, the catheter delivery system 200 can transition from an open configuration of Fig. 7A to the closed configuration of Fig. 7B. Turning to Fig. 7B, the outer shaft 250 of the delivery catheter 210 is shown as being moved distally toward an optional nosecone 230 of the delivery catheter 210. Stated somewhat differently, the growth stent 100 can be disposed between the inner shaft 240 and the outer shaft 250 when the delivery catheter 210 is in the closed configuration. The outer shaft 250 thereby can provide a protective shell for the growth stent 100 for introduction and advancement through a vessel (or lumen) 300 of a patient.

During implantation, the delivery catheter 210 of the catheter delivery system 200 can track through the vasculature with the growth stent 100 and stent expansion system 260 covered by the outer shaft 250. The delivery catheter 210 thereby can be advanced through the vessel (or lumen) 300 of a patient until the end portion 220 reaches an implantation site 310, such as a narrowed lesion or other diseased area, as illustrated in Fig. 8A. Upon becoming disposed adjacent to the implantation site 310, the catheter delivery system 200 can be provided in an open configuration as illustrated in Fig. 8B. Stated somewhat differently, the catheter delivery system 200 can transition from the closed configuration of Fig. 7B to the open configuration of Fig. 8B.

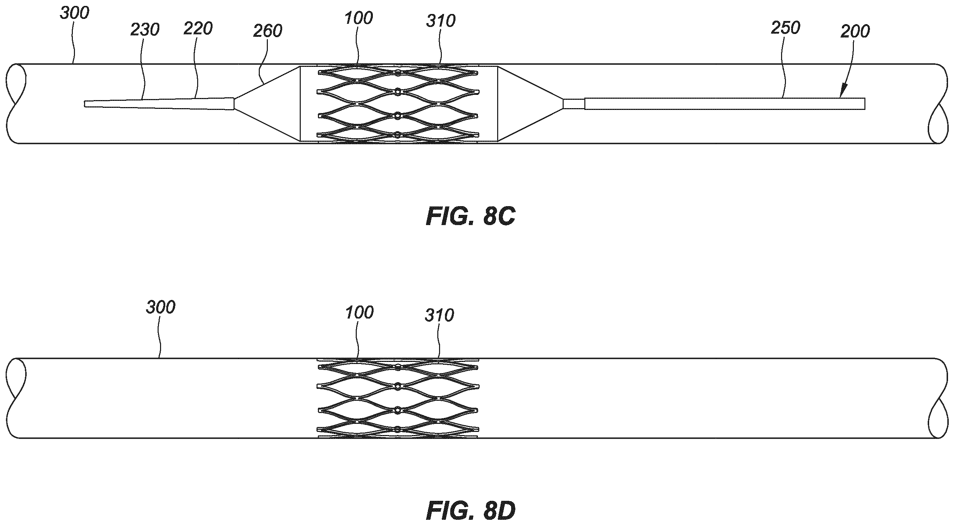

In the open configuration, the outer shaft 250 of the delivery catheter 210 can be disposed proximally or away from the nosecone 230. The delivery catheter 210 thereby can expose the growth stent 100 to the implantation site 310. With the growth stent 100 adjacent to the implantation site 310, the stent expansion system 260 can be activated to expand the growth stent 100 from the initial (or unexpanded or crimped) state as shown in Fig. 8B to the first expanded state of Fig. 8C. In selected embodiments, the stent expansion system 260 can include a balloon that can be inflated to expand the growth stent 100.

In selected embodiments, the balloon can be heat-shaped into any of various shapes to accomplish various inflations and design characteristics. The balloon, for example, can be heat-shaped by heating the balloon in a suitable mold while the balloon is under pressure, thereby forming the balloon into a desired shape. Additionally and/or alternatively, the balloon may be heat-shaped to include one or more proximal and/or distal shoulders (not shown) in the crimped state. The shoulders can provide an area with a diameter that is greater than the area of the balloon upon which the growth stent 100 is crimped. Advantageously, the shoulders can help keep the growth stent 100 in a proper position when the delivery catheter 210 is unsheathed and/or when moving while exposed throughout the vasculature.

The shoulders alternatively can be made of plastic or other materials beneath the balloon. Exemplary other materials may include foams, acrylics, and/or mechanized objects, without limitation. The balloon may be optimized to ensure fluid is able to cross beneath the growth stent 100 even at the most minimized diameters of crimp. Optimization of the balloon be accomplished by adjusting the heat-set on the balloon. The proximal and/or distal shoulders that are heat-set to the balloon can be provided with differing sizes to allow for different fluid pathways. A thickness of the balloon optionally can be adjusted for compliance. Decreasing the thickness of the balloon can result in a balloon that may rupture at lower pressures, but increases the compliance to provide a larger inflation diameter range for the growth stent 100. Patients with congenital heart disease may require a large range of inflation diameters on the balloon in the manner discussed herein.

Additionally and/or alternatively, the growth stent 100 optionally can be expanded by expanding a distal portion of the growth stent 100 while a proximal portion of the growth stent 100 is anchored to the delivery catheter 210 without use of the stent expansion system 260. Once the distal portion of the growth stent 100 is expanded, the proximal portion of the growth stent 100 can be disengaged from the delivery catheter 210 and expanded into the lumen 300. The proximal portion of the growth stent 100, for example, may be expanded all at once or in stages.

In the first expanded state, the growth stent 100 can have a first predetermined expanded size, shape, diameter, cross-section and/or other dimension in the manner set forth in more detail above with reference to Fig. 1A. The stent expansion system 260 can be deactivated, retracting to an initial size. If the stent expansion system 260 includes the balloon, the balloon can be deflated. The delivery catheter 210 then can be withdrawn from the lumen 300 once the growth stent 100 in the first expanded state is successfully deployed at the implantation site 310 as shown in Fig. 8D.

The growth stent 100 thereby can be implanted with its functional size at the implantation site 310. As needed, the growth stent 100 can be further expanded to a larger size, such as a teen size and/or adult size, a later date. In a manner similar to the implantation, for example, another delivery catheter 210 can track through the vasculature of the patient and be advanced through the vessel 300 until the end portion 220 reaches the growth stent 100 at the implantation site 310. A stent expansion system 260 of the delivery catheter 210 can be disposed within the internal channel 120 (shown in Fig. 1A) of the growth stent 100 and activated to expand the growth stent 100 from the first expanded state of Fig. 8C to a second expanded state of Fig. 1B.

As discussed earlier herein, the growth stent 100 advantageously can maintain radial strength and performance at all diameters within the expansion range and may be increased in diameter utilizing one or more further delivery catheters 210 to any suitable diameter over the lifetime of the patient. The growth stent 100 may be re-expanded many times throughout the lifetime of the patient depending on the somatic growth of the vasculature. In some embodiments, the growth stent 100 may be re-expanded three times during the life of the patient. In other patients, the growth stent can be re-expanded up to twenty times or more. Stated somewhat differently, the growth stent 100 advantageously may be re-expanded any number of times, from 1 to 20 and beyond.

In the second expanded state, the growth stent 100 can have a second predetermined expanded size, shape, diameter, cross-section and/or other dimension, which is greater than the first predetermined expanded size, shape, diameter, cross-section and/or other dimension of the growth stent 100 in the first expanded state, in the manner set forth in more detail above with reference to Fig. 1B. The stent expansion system 260 can be retracted, and the delivery catheter 210 can be withdrawn from the lumen 300 once the growth stent 100 in the second expanded state is successfully deployed at the implantation site 310 in the manner set forth above with reference to Fig. 8D.

The growth stent 100 advantageously can be implanted in a pediatric patient and expanded as needed throughout the lifetime of the patient in the manner discussed herein. Once delivered and implanted at a certain diameter, the growth stent 100 can be expanded to a larger size at a later time. This applies in the case that the vessels initially receiving one embodiment of the growth stent 100 are continuing to grow, such as throughout the growth of the patient from infancy to adolescence and adulthood. Because the growth stent 100 can have proper strength over all applicable ranges of growth, a separate balloon or other stent expansion system 260 at a later point in the growth cycle of the patient can be tracked to the growth stent 100 at the implantation site 310. This further expansion of the growth stent 100 can occur as necessary for the growth of an individual patient.

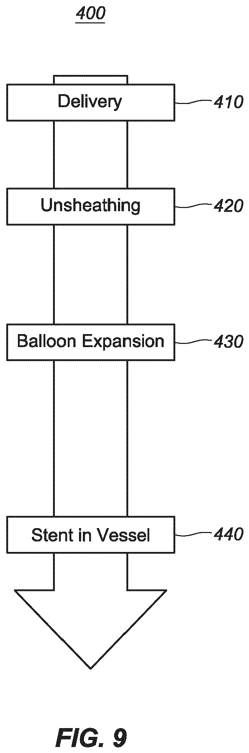

In selected embodiments, the delivery catheter 210 can comprise a balloon catheter with a balloon 260 for expanding the growth stent 100. The balloon catheter can introduce and deploy the growth stent 100 in a manner analogous to the manner by the delivery catheter 210 is described as introducing and deploying the growth stent 100 with reference to Figs. 8A-D. Turning to Fig. 9, for example, an exemplary method 400 for implanting the growth stent 100 (shown in Figs. 8A-D) via a balloon catheter system is shown. The method 400 includes delivering the growth stent 100 to the implantation site 310 of the vessel (or lumen) 300 of a patient (collectively shown in Figs. 8A-D), at 410. The balloon catheter can be advanced through the vessel (or lumen) 300 of a patient until reaching the implantation site 310.

Upon becoming disposed adjacent to the implantation site 310, the balloon catheter can be provided in an open configuration, or unsheathed, at 420, exposing the growth stent 100 to the implantation site 310. Stated somewhat differently, the catheter delivery system 200 can transition from the closed configuration of Fig. 7B to the open configuration of Fig. 8B. The balloon 260 can be filled with fluid and thereby expand in size, at 430. Being disposed in the internal channel 120 (shown in Figs. 1A-B) of the growth stent 100, the expanding balloon 260 can expand the growth stent 100 from the initial (or unexpanded or crimped) state as shown in Fig. 8B to the first expanded state of Fig. 8C.

The growth stent 100 in the first expanded state thus can engage, and/or become embedded into, a wall of the vessel 300 at the implantation site 310, at 440. The balloon 260 then can be re-compressed and removed from the patient's body, leaving the growth stent 100 engaged with the vessel 300. In other words, the growth stent 100 can be introduced in the initial state and, once expanded by the balloon 260, can hold the vessel 300 open without the support of the balloon 260 after the balloon dilation. The growth stent 100 in the first expanded state thereby can be successfully deployed at the implantation site 310.

Additionally and/or alternatively, the balloon catheter can include the optional nosecone 230 (shown in Figs. 7A-B). The nosecone 230 can aid with transitions and trackability through the vessel 300 and serve as a containing end region for the balloon 260 itself. The nosecone 230 can be designed using similar plastics as the shafts 240, 250, often with a triangular shape to aid with transitions and tracking through vessels 300. The balloon 260 advantageously can be designed with a thin plastic wall that is able to be compressed to fit inside of the delivery catheter 210 and ensure the profile is small enough to be inserted in the desired patient's vasculature. The balloon 260 can be attached to the inner plastic shaft 240 and sealed on the distal end. The inner lumen of the plastic shaft 240 can channel inserted fluid, such as saline, from the proximal end of the shaft to the balloon 260. As the balloon 260 fills with liquid, the balloon 260 can expand to the designed diameter.

When the growth stent 100 is crimped onto the balloon 260, the balloon 260 may be compressed to small diameters and without built in shoulders to hold the growth stent 100 in place. When retracting an outer shaft 250 to expose the growth stent 100 on the balloon 260, the balloon 260 may not have enough resistance to maintain the positioning of the growth stent 100 on the balloon 260. In certain embodiments, retracting the outer shaft 250 to expose the growth stent 100 may cause the outer shaft 250 to latch onto the growth stent 100 and pull the growth stent 100 off of the balloon 260.

In some embodiments of the balloon catheter, this issue may be mitigated through the use of a separate catheter (not shown) disposed between the balloon shaft and the outer shaft 250. This middle catheter may be utilized as a back stop for the growth stent 100 during outer shaft retraction. In this embodiment, the outer shaft 250 can be retracted over the growth stent 100 and the middle catheter as the middle catheter is fixed in relation to the balloon catheter, keeping the growth stent 100 in place during exposition. After the growth stent 100 and middle catheter are exposed, the middle catheter may then be retracted from over the balloon 260 to allow for expansion. This ensures that the growth stent 100 remains crimped in the proper position for balloon expansion at the target location 310 in the body of the patient.

In another alternative embodiment of the balloon catheter, instead of a back stop for the growth stent 100, the middle catheter can overlap with the growth stent 100, creating a sock-like attachment to hold the growth stent 100 in place. This thin sock-like catheter can maintain stent positioning during outer shaft 250 retraction and allow for proper balloon 260 inflation. In some embodiments this sock-like catheter can be retracted before balloon 260 expansion if it overlaps with the growth stent 100. In other alternative embodiments, the sock-like catheter may only overlap the balloon 260 and may be expanded with the balloon 260 and removed with the balloon catheter.

In embodiments of the catheter delivery system 200 that contain the growth stent 100 crimped onto the balloon 260, the growth stent 100 can expand with the balloon 260 because of the radial pressure from the filled liquid. As liquid is removed from the balloon 260, back pressure can bring the balloon 260 back to its original compressed state, leaving the expanded growth stent 100 separated from the balloon 260 and the balloon catheter. In one embodiment, the balloon catheter may have a handle on the proximal ends of the shafts 240, 250 to connect the shafts 240, 250, seal the lumens to prevent blood leak and/or provide an ergonomic grip for an operator. The handle can be made of a hard plastic, soft silicone, and/or other solid materials. The embodiment may also contain an inner-most lumen to track over a guidewire (not shown) in the vasculature. The guidewire can provide a tracking rail to ensure the balloon catheter reaches the target location 310 and can be inserted into a lumen on the delivery system.

In selected embodiments, the delivery catheter 210 is advanced to the target location 310 by way of a femoral vein or artery, depending on the endpoint. Other vessels 300 in the patient may be utilized to properly track the delivery catheter 210 to the target location 310. The transitions in the material of the delivery catheter 210 allow proper trackability to the target location 310 despite difficult anatomy.

Additionally and/or alternatively, the catheter delivery system 200 can optionally flush air or fluid out of the delivery catheter 210 before the delivery catheter 210 is introduced into the body of the patient to prevent the addition of air molecules into the bloodstream that may cause embolization and other issues in the vessel 300. Flushing air out of a delivery catheter 210 may include, for example, flushing air out of the delivery catheter 210 with pressurized fluid. The catheter delivery system 200 can include the delivery catheter 210 and one or more inner lumens (not shown) at least partially disposed within the delivery catheter 210. The inner lumens can define at least one opening for a guidewire (not shown) and at least one opening connected to a balloon. Fluid can be introduced into the inner lumens in the

43     **EP 4 684 766 A1**     44

opening for a guidewire with at least some of the introduced fluid exiting the inner lumens through a distal opening to remove the air from the delivery catheter 210.

The sizing of the delivered growth stent 100 can allow the growth stent 100 to be safer for neonates and other younger patients. The growth stent 100, in one embodiment, can be delivered at an outer diameter of less than 2.5 millimeters when in a crimped state, while allowing for later expansion to over 20 millimeters in outer diameter in a fully expanded state.

The forgoing primarily describes embodiments of the growth stent 100 that are balloon-expandable for purposes of illustration only, not for purposes of limitation. It will be appreciated, however, that the catheter delivery system 200 shown and described herein can be readily modified for delivery of self-expandable growth stents, prosthetic heart valves and/or other medical devices. In other words, delivering self-expandable growth stents to an implantation location can be performed percutaneously using modified versions of the delivery devices of the present disclosure. In general terms, an exemplary modified version of the delivery devices can include a transcatheter assembly (not shown) with a delivery sheath and/or additional sheaths as described above. The devices generally further include a delivery catheter, a balloon catheter, and/or a guide wire.

In other embodiments, the frame of the stent or valve may be a self-expanding material. In these embodiments, the delivery catheter does not require a balloon as the frame will expand back to the shape it was set. The delivery catheter 200 for self-expanding stents may simply unsheathe the outer lumen to expose the frame, having it take the shape of the set.

Current market growth stents are unable to cover the total range necessary for the lifetime of the lesion in the patient, specifically a pediatric patient with congenital or anatomical disease. In this case, the growth stent may start small enough to be delivered safely into a neonate but is unable to grow to the adult size. Conversely, a growth stent that expands to large enough for the adult lesion cannot be crimped small enough to be safely implanted in a neonate on a catheter, as neonatal vessels are considerable smaller than an adult vessel.

Varied congenital disease states can be defined as narrowed lesions, such as Tetralogy of Fallot, Aortic Coarctation, various atresia, and various stenosis.

A further understanding of the nature and advantages of the growth stent 100 will become apparent by reference to the remaining portions of the specification and drawings. Variations and the optional features noted above may be added to embodiments of the growth stent 100, either alone or in various combinations, as appropriate. Specific numerical values for sizes, shapes, diameters, cross-sections or other dimensions are set forth herein for purposes of illustration only and not for purposes of limitation.

The described embodiments are susceptible to various modifications and alternative forms, and specific examples thereof have been shown by way of example in the drawings and are herein described in detail. It should be understood, however, that the described embodiments are not to be limited to the particular forms or methods disclosed, but to the contrary, the present disclosure is to cover all modifications, equivalents, and alternatives.

## Claims

1.  A catheter-insertable and re-expandable flexible growth stent for implantation in a patient, comprising:

    an elongated flexible stent frame comprising first, second and third annular frame strut arrangements being disposed in axial alignment between a proximal end region and a distal end region of said flexible stent frame,
    the first annular frame strut arrangement having a first zigzag arrangement of first frame strut members with first peaks and first valleys,
    the second annular frame strut arrangement having a second zigzag arrangement of second frame strut members with second peaks and second valleys,
    the third annular frame strut arrangement being disposed between the first and second annular frame strut arrangements,
    the third annular frame strut arrangement having a third zigzag arrangement of third frame strut members with:

      a third peak being directly coupled with a corresponding one of the first valleys to form a first strut junction;
      a fourth peak being disposed adjacent to a corresponding one of the first valleys to define a first intermediate gap;
      a third valley being directly coupled with a corresponding one of the second peaks to form a second strut junction; and
      a fourth valley being disposed adjacent to a corresponding one of the second peaks to define a second intermediate gap,

    each of the third frame strut members associated with the first strut junction having proximal and distal end regions with respective cross-sections that are less than a cross-section of a central region of the third frame strut member,
    each of the first frame strut members associated with the first strut junction having proximal and distal end regions with respective cross-sections that are less than a cross-section of a central region of the first frame strut member,
    each of the third frame strut members associated with the second strut junction having proximal and distal end regions with respective cross-sections that are less than a cross-section of a central region of the third frame strut member,

each of the second frame strut members associated with the second strut junction having proximal and distal end regions with respective cross-sections that are less than a cross-section of a central region of the second frame strut member,

wherein the first and second intermediate gaps enhance an axial flexibility of said flexible stent frame,

wherein said flexible stent frame has an initial cross-section for facilitating implantation within a selected lumen of the patient, said flexible stent frame being configured to expand from the initial cross-section to a first stable expanded state for supporting the selected lumen at implantation, said flexible stent frame in the first stable expanded state defining an internal channel with a first cross-section, and

wherein said flexible stent frame is configured to subsequently expand from the first stable expanded state to a second stable expanded state as the selected lumen grows while maintaining the axial flexibility of said flexible stent frame, said flexible stent frame in the second stable expanded state continuing to support the selected lumen and defining the internal channel with a second cross-section being larger than the first cross-section.

2. The growth stent of claim 1, wherein each third peak of the zigzag arrangement of the third frame strut members is coupled with a respective first valley of the zigzag arrangement of the first frame strut members to form respective first strut junctions, and wherein each third valley of the zigzag arrangement of the third frame strut members is coupled with a respective second peak of the zigzag arrangement of the second frame strut members to form respective second strut junctions.

3. The growth stent of claim 1 or claim 2, wherein at least one of the first and second strut junctions comprises an arcuate member for providing the axial flexibility of said flexible stent frame.

4. The growth stent of claim 3, wherein the arcuate member is configured to provide a lever point for enabling said flexible stent frame to bend during implantation within the selected lumen.

5. The growth stent of claim 4, wherein the arcuate member comprises a c-shaped member that is configured to collapse upon itself for providing the bend in said flexible stent frame.

6. The growth stent of claim 4 or claim 5, wherein the arcuate member comprises a c-shaped member that is configured to open into a straighten member for providing the bend in said flexible stent frame.

7. The growth stent of any one of claims 1-6, wherein said flexible stent frame is made from cobalt chromium.

8. The growth stent of any one of claims 1-7, further comprising a covering for enclosing a periphery of said flexible stent frame.

9. The growth stent of claim 8, wherein said covering comprises a fluid-impermeable covering for preventing leaks due to ingrowth of the selected lumen as the patient grows.

10. The growth stent of any one of claims 1-9, wherein the initial first cross-section is less than or equal to eight millimeters.

11. The growth stent of any one of claims 1-10, wherein the first cross-section is between eight millimeters and ten millimeters.

12. The growth stent of any one of claims 1-11, wherein the wherein the second cross-section is between fifteen millimeters and twenty millimeters.

FIG. 1A

**FIG. 1B**

*100*

*100*

110

110

D

120

2mm

8mm

**FIG. 2A**

**FIG. 2B**

*100*

D

*120*

*110*

20mm+

*FIG. 2D*

*110*

*100*

*120*

D

15mm

*FIG. 2C*

28

FIG. 3A

FIG. 3B

_100_

**FIG. 4A**

FIG. 4B

**FIG. 5B**

**FIG. 5A**

FIG. 5C

**FIG. 5D**

EP 4 684 766 A1

FIG. 6B

FIG. 6A

**FIG. 7A**

**FIG. 7B**

EP 4 684 766 A1

**FIG. 8A**

**FIG. 8B**

EP 4 684 766 A1

**FIG. 8C**

**FIG. 8D**

_400_

```
┌─────────────────┐
│    Delivery     │──── 410
└─────────────────┘
         │
┌─────────────────┐
│   Unsheathing   │──── 420
└─────────────────┘
         │
┌─────────────────┐
│ Balloon Expansion │──── 430
└─────────────────┘
         │
┌─────────────────┐
│  Stent in Vessel │──── 440
└─────────────────┘
         ▼
```

**FIG. 9**

Fig. 10A

Fig. 10B

EP 4 684 766 A1

Fig. 11A

EP 4 684 766 A1

Fig. 11B

Fig. 12

Fig. 13A

EP 4 684 766 A1

Fig. 13B

**EUROPEAN SEARCH REPORT**

Europäisches
Patentamt
European
Patent Office
Office européen
des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2020/176122 A1 (RENATA MEDICAL INC [US]) 3 September 2020 (2020-09-03) * paragraphs [0026], [0058], [0059], [0065], [0085], [0092]; figure 4A * | 1-12 | INV. A61F2/915 ADD. A61F2/07 A61F2/958 |
| Y | US 2024/189092 A1 (MARSHALL COREY [US] ET AL) 13 June 2024 (2024-06-13) * paragraph [0108]; figure 3A and 3B * | 1-12 | |
| Y | US 2012/172972 A1 (MEYER MICHAEL P [US] ET AL) 5 July 2012 (2012-07-05) * paragraph [0040]; figure 2B * | 1-12 | |
| A | EP 2 656 818 A1 (LIFETECH SCIENT SHENZHEN CO [CN]) 30 October 2013 (2013-10-30) * paragraph [0031]; figure 4 * | 1-12 | |
| A | US 2009/105809 A1 (LEE MICHAEL J [US] ET AL) 23 April 2009 (2009-04-23) * paragraph [0223]; figure 1B * | 1-12 | |

TECHNICAL FIELDS
SEARCHED (IPC)

A61F

The present search report has been drawn up for all claims

| Place of search | Date of completion of the search | Examiner |
|---|---|---|
| The Hague | 24 October 2025 | Stratakos, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 18 0521

This annex lists the patent family members relating to the patent documents cited in the above-mentioned European search report.
The members are as contained in the European Patent Office EDP file on
The European Patent Office is in no way liable for these particulars which are merely given for the purpose of information.

24-10-2025

| Patent document cited in search report | | Publication date | Patent family member(s) | | Publication date |
|---|---|---|---|---|---|
| WO 2020176122 | A1 | 03-09-2020 | AU | 2019431385 A1 | 02-09-2021 |
| | | | AU | 2022201168 A1 | 17-03-2022 |
| | | | CA | 3131660 A1 | 03-09-2020 |
| | | | CL | 2021002160 A1 | 28-01-2022 |
| | | | CL | 2022000519 A1 | 30-09-2022 |
| | | | CN | 113490470 A | 08-10-2021 |
| | | | EA | 202192201 A1 | 13-10-2021 |
| | | | EP | 3930636 A1 | 05-01-2022 |
| | | | EP | 4467112 A2 | 27-11-2024 |
| | | | ES | 3015262 T3 | 30-04-2025 |
| | | | IL | 285696 A | 31-10-2021 |
| | | | IL | 290483 A | 01-04-2022 |
| | | | IL | 305385 A | 01-10-2023 |
| | | | JP | 2022107060 A | 20-07-2022 |
| | | | JP | 2022514441 A | 10-02-2022 |
| | | | JP | 2023016920 A | 02-02-2023 |
| | | | JP | 2023171572 A | 01-12-2023 |
| | | | JP | 2023171573 A | 01-12-2023 |
| | | | KR | 20210122892 A | 12-10-2021 |
| | | | KR | 20220027258 A | 07-03-2022 |
| | | | PT | 3930636 T | 31-01-2025 |
| | | | US | 10702407 B1 | 07-07-2020 |
| | | | US | 2020276037 A1 | 03-09-2020 |
| | | | US | 2021085496 A1 | 25-03-2021 |
| | | | US | 2022168123 A1 | 02-06-2022 |
| | | | US | 2023085236 A1 | 16-03-2023 |
| | | | US | 2024065866 A1 | 29-02-2024 |
| | | | US | 2024382326 A1 | 21-11-2024 |
| | | | WO | 2020176122 A1 | 03-09-2020 |
| US 2024189092 | A1 | 13-06-2024 | AU | 2023390347 A1 | 19-06-2025 |
| | | | AU | 2025208476 A1 | 14-08-2025 |
| | | | EP | 4629933 A1 | 15-10-2025 |
| | | | EP | 4640189 A2 | 29-10-2025 |
| | | | IL | 321224 A | 01-07-2025 |
| | | | US | 12004939 B1 | 11-06-2024 |
| | | | US | 2024261083 A1 | 08-08-2024 |
| | | | US | 2025114185 A1 | 10-04-2025 |
| | | | US | 2025288407 A1 | 18-09-2025 |
| | | | WO | 2024124034 A1 | 13-06-2024 |
| US 2012172972 | A1 | 05-07-2012 | EP | 2658484 A1 | 06-11-2013 |
| | | | JP | 2014508559 A | 10-04-2014 |
| | | | US | 2012172972 A1 | 05-07-2012 |
| | | | WO | 2012091769 A1 | 05-07-2012 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 25 18 0521

This annex lists the patent family members relating to the patent documents cited in the above-mentioned European search report.
The members are as contained in the European Patent Office EDP file on
The European Patent Office is in no way liable for these particulars which are merely given for the purpose of information.

24-10-2025

| Patent document cited in search report | | Publication date | Patent family member(s) | | Publication date |
|---|---|---|---|---|---|
| EP 2656818 | A1 | 30-10-2013 | CN | 102525701 A | 04-07-2012 |
| | | | EP | 2656818 A1 | 30-10-2013 |
| | | | US | 2013325102 A1 | 05-12-2013 |
| | | | WO | 2012083796 A1 | 28-06-2012 |
| US 2009105809 | A1 | 23-04-2009 | EP | 2231080 A1 | 29-09-2010 |
| | | | EP | 3025685 A1 | 01-06-2016 |
| | | | EP | 3505142 A1 | 03-07-2019 |
| | | | US | 2009105809 A1 | 23-04-2009 |
| | | | US | 2012197384 A1 | 02-08-2012 |
| | | | US | 2016346105 A1 | 01-12-2016 |
| | | | US | 2019254847 A1 | 22-08-2019 |
| | | | WO | 2009051607 A1 | 23-04-2009 |

EPO FORM P0459

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 12004939 B